# EUROPEAN PATENT APPLICATION

(11) **EP 4 239 062 A1**
(43) Date of publication of application: **06.09.2023**
(21) Application number: 21886394.2
(22) Date of filing: 29.10.2021
(51) Int. Cl.: C12N 5/10, A61K 35/17, A61P 35/02, C12N 15/62, C12N 15/90

(54) **CXCL12-RECEPTOR-EXPRESSING CHIMERIC ANTIGEN RECEPTOR (CAR)-T CELL**

(30) Priority: 29.10.2020 US 202063107027 P
(71) Applicant: RIKEN, Wako-shi, Saitama 351-0198 (JP)
(72) Inventor: ISHIKAWA, Fumihiko, Wako-shi, Saitama 351-0198 (JP); SAITO, Yoriko, Wako-shi, Saitama 351-0198 (JP); ITOH, Ari, Wako-shi, Saitama 351-0198 (JP); SHULTZ, Leonard D., Bar Harbor, Maine 04609 (US)
(74) Representative: Mewburn Ellis LLP
(86) International application number: PCT/JP2021/040069
(87) International publication number: WO 2022/092277

(57) **Abstract**

This invention provides, as a therapeutic method for eradication of neoplastic diseases of the blood with poor diagnosis, a cell co-expressing a chimeric antigen receptor (CAR) protein and a CXCL12 receptor protein on the cell membrane, and an agent and a pharmaceutical composition having anti-tumor activity, which comprises such cell.

## Description

### Technical Field

The present invention relates to an improved chimeric antigen receptor (CAR)-T cell therapy. More specifically, the present invention relates to a modified cell co-expressing a chimeric antigen receptor and a CXCL12 receptor protein, and an agent and a pharmaceutical composition having anti-tumor activity which comprise such cell. Further, the present invention relates to a method for the treatment of neoplastic diseases with the use of a modified cell co-expressing a chimeric antigen receptor and a CXCL12 receptor protein.

### Background Art

Chimeric antigen receptor (CAR)-T cell therapy is a method of adoptive immunotherapy using a T cell expressing CAR (CAR-T cell) that targets a particular antigen expressed on a cell surface, such as a tumor cell. The CAR-T cell is designed to recognize a target antigen to attack a cell expressing the target antigen.

In recent years, a CAR-T cell targeting CD19 has emerged as a promising treatment modality of choice for relapsed and refractory B-cell acute lymphatic leukemia (B-ALL) and diffuse large B-cell lymphoma (DLBCL) (Non-Patent Literatures 1 to 3), and such CAR-T cell is known to be capable of exerting satisfactory therapeutic effects by single-dose administration thereof.

Much effort has heretofore been made to evaluate whether CAR-T cell therapy can be applied to other hematologic malignancies and solid tumors. Among hematologic malignancies, for example, acute myeloid leukemia (AML) has been showing dismal clinical outcomes. To prolong survival of AML patients, CAR- T cells targeting molecules such as CD33 (Non-Patent Literature 4), CD123 (Non-Patent Literature 5), and tumor-associated antigen Lewis-Y (TAA-LeY) (Non-Patent Literature 6), have been developed and under clinical evaluation.

### Prior Art Literatures

### Non-Patent Literatures

Non-Patent Literature 1: Grupp, S.A., Kalos, M., Barrett, D., Aplenc, R., Porter, D.L., Rheingold, S.R., Teachey, D.T., Chew, A., Hauck, B., Wright, J.F., et al., 2013, Chimeric antigen receptor-modified T cells for acute lymphoid leukemia, N. Engl. J. Med., 368, 1509-1518. 10.1056/NEJMoa1215134.
Non-Patent Literature 2: Lee, D.W., Kochenderfer, J.N., Stetler-Stevenson, M., Cui, Y.K., Delbrook, C., Feldman, S.A., Fry, T.J., Orentas, R., Sabatino, M., Shah, N.N., et al., 2015, T cells expressing CD19 chimeric antigen receptors for acute lymphoblastic leukaemia in children and young adults: a phase 1 dose-escalation trial, Lancet 385, 517-528. 10.1016/S0140-6736(14)61403-3.
Non-Patent Literature 3: Park, J.H., Riviere, I., Gonen, M., Wang, X., Senechal, B., Curran, K.J., Sauter, C., Wang, Y., Santomasso, B., Mead, E., et al., 2018, Long-Term Follow-up of CD19 CAR Therapy in Acute Lymphoblastic leukemia, N. Engl. J. Med., 378, 449-459. 10.1056/NEJMoa1709919.
Non-Patent Literature 4: Kenderian, S.S., Rosado, F.G., Sykes, D.B., Hoyer, J.D., and Lacy, M.Q., 2015, Long-term complete clinical and hematological responses of the TEMPI syndrome after autologous stem cell transplantation, Leukemia 29, 2414-2416. 10.1038/leu.2015.298. Non-Patent Literature 5: Mardiros, A., Forman, S.J., and Budde, L.E., 2015, T cells expressing CD123 chimeric antigen receptors for treatment of acute myeloid leukemia, Curr. Opin. Hematol. 22, 484-488. 10.1097/MOH.0000000000000190.
Non-Patent Literature 6: Ritchie, D.S., Neeson, P.J., Khot, A., Peinert, S., Tai, T., Tainton, K., Chen, K., Shin, M., Wall, D.M., Honemann, D., et al., 2013, Persistence and efficacy of second generation CAR T cell against the LeY antigen in acute myeloid leukemia, Mol. Ther. 21, 2122-2129. 10.1038/mt.2013.154.

### Summary of the Invention

### Objects to Be Attained by the Invention

There are several major obstacles in developing CAR-T cells for AML. First, gene expression is similar between AML cells and normal hematopoietic stem/progenitor cells. Thus, it is difficult to find cell surface molecules as appropriate targets for CAR-T cells.

Second, elimination of CD19+ B lymphocytes can be managed by intravenous immunoglobulin supplementation. However, CD33 as mentioned above is a molecule expressed by hematopoietic stem/progenitor cells (HSPCs) and neutrophils, and, because of potential adverse effects against normal myeloid cells such as neutrophils, CD33-targeted therapy is likely to cause occasionally-fatal febrile neutropenia and opportunistic infection by fungi and bacteria.

Finally, although CD19 is expressed by every B-ALL cell of virtually all B-ALL patients, the expression of cell surface molecules in AML is highly heterogeneous across and within patients.

### Means for Attaining the Objects

We performed global transcriptome analysis using leukemia-initiating cells (LICs), normal CD34+CD38-CD45RA-hematopoietic stem cells (HSCs), and neutrophils from clinically-aggressive AML patients and also analyzed the gene expression in AML-initiating cells from patients that were verified to cause AML through *in vivo* NOD/SCID/Il2rgKO (NSG) xenograft assays.

As a result, we identified CD25 (IL-2 receptor alpha chain, IL2RA) as a potential therapeutic cell surface molecule in AML (Saito et al., STM, 2010). CD25/IL2RA has been reported as a marker for poor prognosis in AML (Gonen et al., Blood, 2012). In addition, CD25/IL2RA is expressed in other hematologic malignancies, such as chronic myeloid leukemia (CML), adult T cell leukemia/lymphoma, and Hodgkin's lymphoma. However, CD25 expression is also observed in regulatory T cells and a sub-population of activated T cells. This complicates evaluation of appropriateness of CD25/IL2RA as a target molecule in CAR-T cell therapy.

To develop CAR-T cell therapy for poor-prognosis AML, we constructed a lentiviral vector containing a TCR-signal sequence and Fab antigen recognition sites for human CD25/IL2RA antigen. CD25-CAR lentiviral particles were transduced into cord blood-derived human T cells, and the expansion of CD25 CAR-T cells to 2 × 10⁷ or more was achieved *in vitro.*

As a result of evaluation of the targeting efficacy of CD25-CAR T cells on primary cultured AML cells with poor prognosis *in vitro,* cytotoxicity was observed only when CD25-expressing AML cells were the targets, and cytotoxicity was not observed on CD25-deficient leukemic cells.

Since antigen-dependent cytotoxicity of CD25 CAR-T cells was confirmed, subsequently, we composed *in vivo* treatment experiments using NOD/SCID/IL2rgKO (NSG) patient-derived xenograft (PDX) animals for AML. As a result of single injection of 5 × 10⁶ CD25-targeting CAR-T cells into 3 mice engrafted with AML cells (CD33-positive blood cells) from an AML patient (U390#2), the number of patient-derived leukemic cells was decreased in the peripheral blood (PB) of 1 out of 3 PDX mice. The 3 mice were sacrificed 4 weeks after injection and observed. The results demonstrated that the therapeutic effects were not sufficient to eliminate aggressive AML cells from the bone marrow of the recipient mice. HE staining demonstrated that the majority of myeloid cells were patient-derived AML cells.

To solve the problem such that eradication of human AML cells is not optimal, we aimed at directing CAR-T cells toward the bone marrow more efficiently. To this end, we further modified human T cells to have expression of CXCR4 known as a homing receptor to induce CD25 CAR-T cells to the bone marrow.

We analyzed the peripheral blood cells harvested from the mice to which CAR-T cells had been administered by flow cytometry. As a result, the CXCR4-expressing CD25 CAR-T cells were found to proliferate more efficiently *in vivo* than CD25 CAR-T cells not expressing CXCR4. In accord with the high frequency and the number of CXCR4-expressing CD25 CAR T cells, the CXCR4-expressing CD25 CAR T cells eradicated human AML cells in peripheral blood, spleen, bone marrow, and liver. With long-term observation experiments, human CXCR4-expressing CD25 CAR-T cells remained in the bone marrow of the treated mice for 4 months or longer, and prevented relapse of AML in the recipient mice. We did not observe serious adverse effect against normal human immunocytes by the CXCR4-expressing CD25 CAR-T cells, such as unexpected proliferation of CAR-T cells, graft-versus-host disease, or serious inflammatory responses, in NSG mice engrafted to have normal human innate and acquired immunity.

We have conducted further studies on the basis of the above-described findings. As a result, we found that improved effects of CAR-T cell therapy attained by CXCR4 co-expression would also be attained by CAR-T cells targeting other molecules. This has led to the completion of the present invention.

Specifically, the embodiments of the present invention are as described below.
1. A cell co-expressing a chimeric antigen receptor (CAR) protein and a CXCL12 receptor protein on the cell membrane.
2. The cell according to 1 above, wherein the CXCL12 receptor is CXCR4.
3. The cell according to 1 or 2 above, wherein the chimeric antigen receptor (CAR) protein targets a cell surface antigen selected from among CD25 (IL-2 receptor alpha chain), CD19, and CD7.
4. The cell according to any of 1 to 3 above, which is a T cell.
5. An agent exhibiting anti-tumor activity, which comprises the cell according to any of 1 to 4 above.
6. The agent according to 5 above, which is used in combination with a further anti-tumor agent and/or anti-tumor therapy.
7. A pharmaceutical composition comprising the agent according to 5 above and a pharmaceutically acceptable carrier.
8. The agent according to 5 or 6 above or the pharmaceutical composition according to 7 above, for the treatment and/or the prevention of relapse of a neoplastic disease selected from the group consisting of acute myeloid leukemia (AML), adult T cell leukemia, B-cell acute lymphatic leukemia (B-ALL), T-cell acute lymphatic leukemia (T-ALL), mixed phenotype acute leukemia (MPAL), chronic myeloid leukemia (CML), Hodgkin's lymphoma, and non-Hodgkin's lymphoma.
9. A method for preparing the cell according to any of 1 to 4 above, which comprises introducing a first polynucleotide encoding a chimeric antigen receptor (CAR) protein and a second polynucleotide encoding a CXCL12 receptor protein into a cell.
10. The method according to 9 above, wherein the chimeric antigen receptor (CAR) protein targets a cell surface antigen selected from among CD25 (IL-2 receptor alpha chain), CD19, and CD7.
11. The method according to 9 or 10 above, wherein the first polynucleotide and the second polynucleotide are introduced into a cell by the same vector or separate vectors.
12. A method for the treatment and/or the prevention of relapse of a neoplastic disease in a subject, which comprises a step of administering a cell co-expressing a chimeric antigen receptor (CAR) protein targeting CD25 (IL-2 receptor alpha chain) and a CXCL12 receptor protein on the cell membrane to the subject.
13. The method according to 12 above, wherein the chimeric antigen receptor (CAR) protein targets a cell surface antigen selected from among CD25 (IL-2 receptor alpha chain), CD19, and CD7.
14. The method according to 12 or 13 above, wherein the neoplastic disease is selected from the group consisting of acute myeloid leukemia (AML), adult T cell leukemia, B-cell acute lymphatic leukemia (B-ALL), T-cell acute lymphatic leukemia (T-ALL), mixed phenotype acute leukemia (MPAL), chronic myeloid leukemia (CML), Hodgkin's lymphoma, and non-Hodgkin's lymphoma.
15. The method according to any of 12 to 14 above, wherein the cell is administered once to a subject.
16. The method according to any of 12 to 15 above, wherein the cell is administered in the range of 10⁴ to 10⁹ cells per kg body weight of the subject.
17. The method according to any of 12 to 16 above, which further comprises a step of measuring the expression level of a target cell surface antigen in a tumor cell of the subject before the step of administering the cell.
18. The method according to any of 12 to 17 above, which further comprises a step of evaluating therapeutic effects after the step of administering the cell.
19. The method according to 18 above, wherein the therapeutic effect is evaluated based on one or more indices selected from (i) decrease in tumor cells in the blood, (ii) decrease in tumor cells in the bone marrow, (iii) decrease in tumor cells in the spleen, and (iv) suppression of tumor cell infiltration into the liver, in the subject.
20. A cell for use in the treatment and/or the prevention of relapse of a cancer, which co-expresses a chimeric antigen receptor (CAR) protein and a CXCL12 receptor protein on the cell membrane.
21. The cell according to 20 above, wherein the chimeric antigen receptor (CAR) protein targets a cell surface antigen selected from among CD25 (IL-2 receptor alpha chain), CD19, and CD7.
22. The cell according to 20 or 21 above, wherein the cancer is a neoplastic disease selected from the group consisting of acute myeloid leukemia (AML), adult T cell leukemia, B-cell acute lymphatic leukemia (B-ALL), T-cell acute lymphatic leukemia (T-ALL), mixed phenotype acute leukemia (MPAL), chronic myeloid leukemia (CML), Hodgkin's lymphoma, and non-Hodgkin's lymphoma.
23. The cell according to any of 20 to 22 above, which is a T cell.

The present description encompasses the contents disclosed in U.S. Provisional Patent Application No. 63/107027 filed on October 29, 2020 on which the priority of the present application is based.

### Advantageous Effects of the Invention

The present invention can provide a very effective method for the treatment and/or the prevention of relapse of poor-prognosis neoplastic diseases that are difficult to completely cure. A method of CXCR4-expressing CAR-T cell therapy is a promising treatment strategy for neoplastic diseases with poor prognosis.

### Brief Description of the Drawings

[Figure 1]

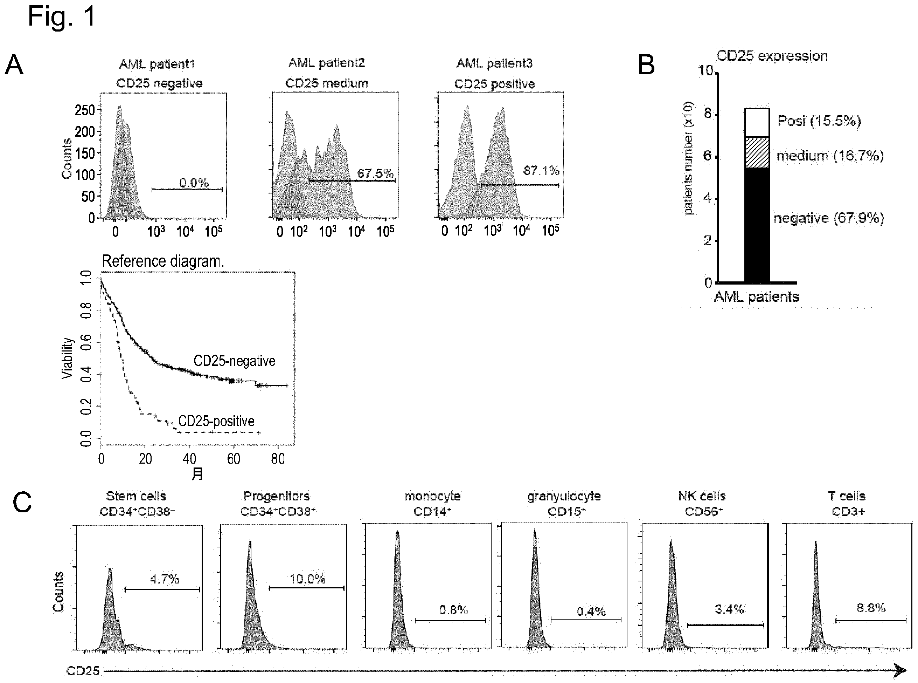

   Fig. 1 A shows proportions of CD25-expressing cells in AML cells in the bone marrow of PDX (patient-derived xenograft) mice engrafted with AML cells derived from 3 patients exhibiting different CD25 expression levels into immunodeficient mice. Charts each show CD25-negative expression, medium-level expression of CD25, and high-level expression of CD25, from left to right. The reference diagram shows survival curves of CD25-negative AML patients and CD25-positive AML patients reported in 2012. CD25 expression has been reported to be associated with poor prognosis of AML (Gonen et al., Blood, 2012). Fig. 1 B shows CD25-positive rates of 84 AML patients determined by flow cytometry. Approximately 30% of AML patients exhibit high-level and medium-level CD25 expressions. Fig. 1 C shows the results of evaluation of CD25 expression in myeloid cells of humanized mice prepared by transplanting cord-blood-derived human hematopoietic stem cells into immunodeficient mice. CD25 is a regulatory T cell marker, and CD25 expression is observed in some activated T cells. In contrast, CD25 expression is not observed in normal monocytes (CD14+), granulocytes (CD15+), and CD34⁺CD38⁻ stem cells.
[Figure 2]

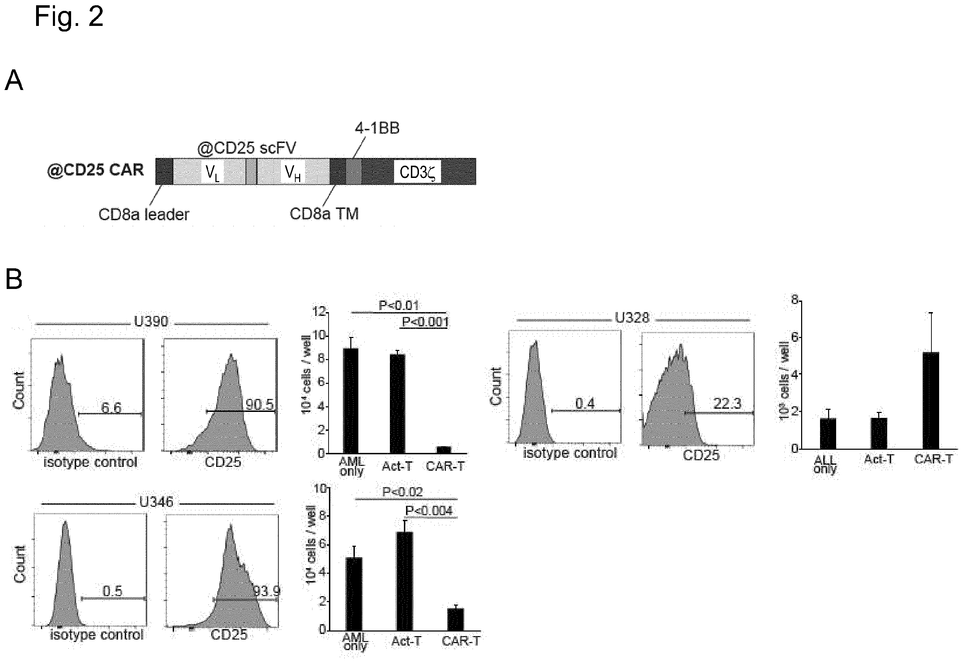

   Fig. 2 A shows an example of a CAR-T construct targeting CD25. This is a second-generation CAR comprising scFV of an anti-CD25 monoclonal antibody as a target binding domain, a CD8a-derived leader sequence, a hinge, a transmembrane domain, and 4-1BB and CD3ζ-derived intracellular domain. Fig. 2 B shows *in vitro* cytotoxic activity of CD25 CAR-T cells against CD25-positive AML cells (U390, U346) and CD25-negative ALL cells (U328). CAR-T cells target CD25-expressing AML cells (U390 and U346) but do not target CD25-negative leukemic cells (U328), exhibiting CD25-dependent cytotoxic activity.
[Figure 3]

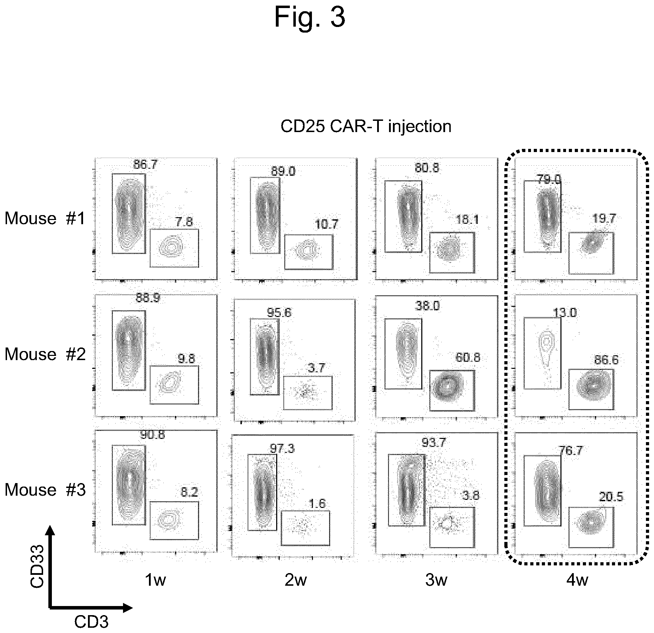

   Fig. 3 shows the results of flow cytometric analysis with the use of CD33 and CD3 expressions as indices concerning human CD45-positive cells by administering CD25 CAR-T cells (5 × 10⁶ cells) to PDX mice engrafted with human AML cells (#1, #2, and #3) and collecting peripheral blood samples once a week for 4 weeks. While increase in T cells (CD3+) and decrease in AML cells (CD33+) were observed in all mice, AML cells were observed to remain in the peripheral blood even 4 weeks later. While satisfactory therapeutic effects were observed in Mouse #2, effects of the CD25-targeting CAR-T cell therapy were found to vary.
[Figure 4]

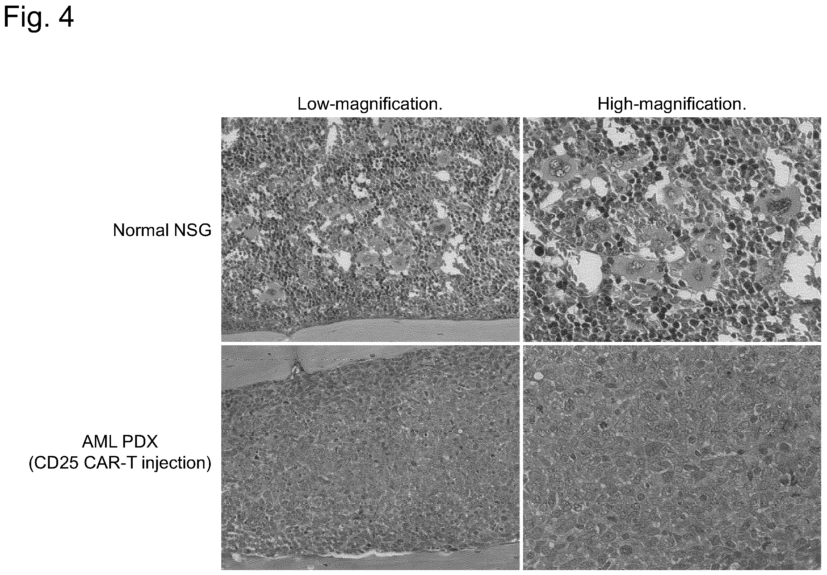

   Fig. 4 shows the results of HE staining of bone marrow sections obtained by administering CD25 CAR-T cells (5×10⁶ cells) to PDX mice engrafted with AML cells and dissecting the mice 4 weeks later. While a variety of cells, such as granulocytes and myelocytes, were observed in the bone marrow of immunodeficient mice (normal NSG), the bone marrow of AML PDX mice were filled with leukemic blasts, and therapeutic effects attained with the use of CD25 CAR-T cells were not sufficient. Left: low-magnification; right: high-magnification.
[Figure 5-1]

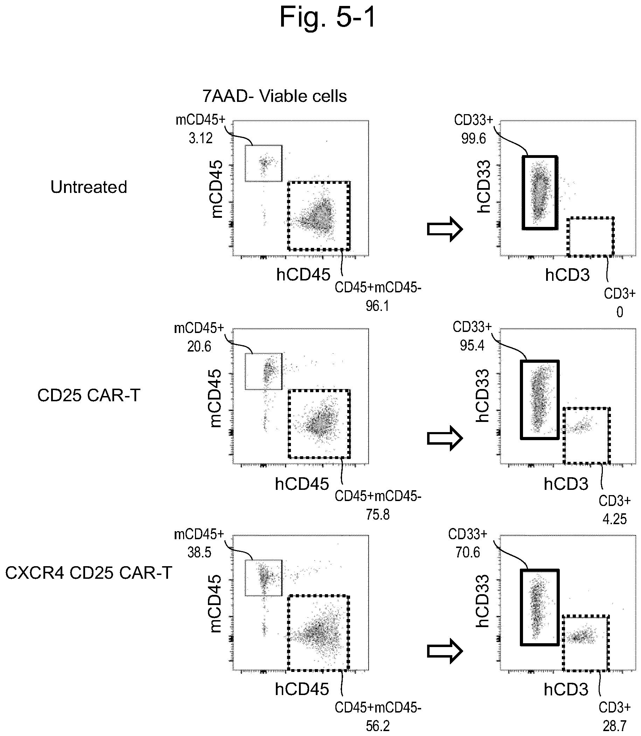

   Fig. 5-1 shows the results of flow cytometry of CD45-positive cells in the peripheral blood obtained 2 weeks after administration of CXCR4-expressing CD25 CAR-T cells to PDX mice engrafted with human AML cells (U390), in comparison with the results without administration of CAR-T cells (untreated) and those by administration of CD25 CAR-T cells. The injected CXCR4-expressing CD25 CAR-T cells were proliferated *in vivo.*
[Figure 5-2]

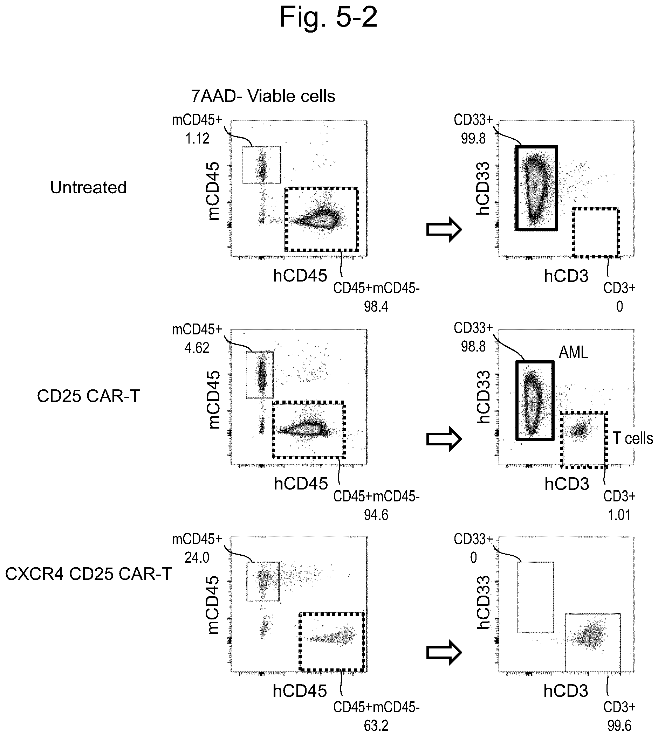

   Fig. 5-2 shows the results of flow cytometry of CD45-positive cells in the peripheral blood obtained 4 weeks after administration of CXCR4-expressing CD25 CAR-T cells to PDX mice engrafted with human AML cells (U390), in comparison with the results without administration of CAR-T cells (untreated) and those by administration of CD25 CAR-T cells. In the case of CD25 CAR-T cell administration, substantially all cells remained in human cells are leukemic cells. In the case of CXCR4-expressing CD25 CAR-T cells administration, in contrast, CD33-positive leukemic (AML) cells completely disappeared.
[Figure 5-3]

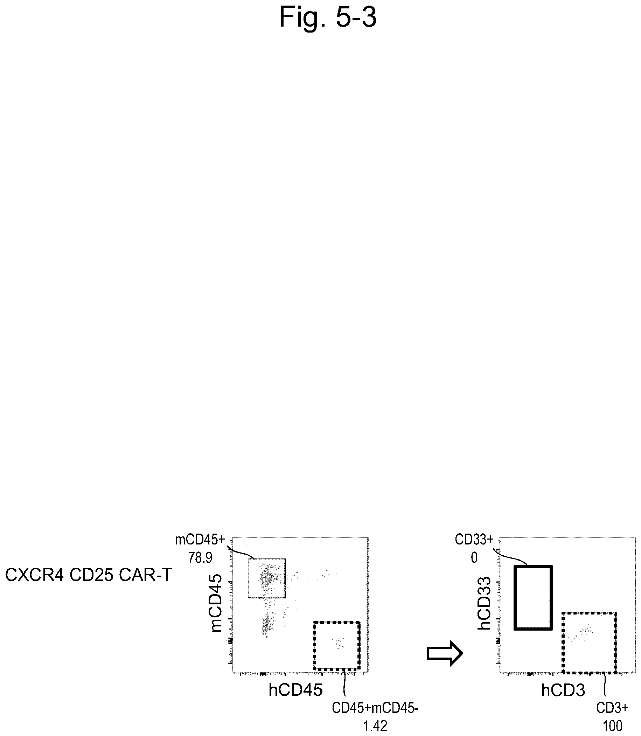

   Fig. 5-3 shows the results of flow cytometry of CD45-positive cells in the peripheral blood obtained 4 months after administration of CXCR4-expressing CD25 CAR-T cells to PDX mice engrafted with human AML cells (U390). AML cells were not detected in the peripheral blood, and the effects of eliminating target AML cells within 4 weeks after administration were maintained for 4 months or longer. After the target cells disappeared, the frequency and the absolute number of CXCR4 CD25 CAR-T cells were gradually reduced, and a majority of the peripheral blood was accounted for by mouse CD45-positive cells.
[Figure 6]

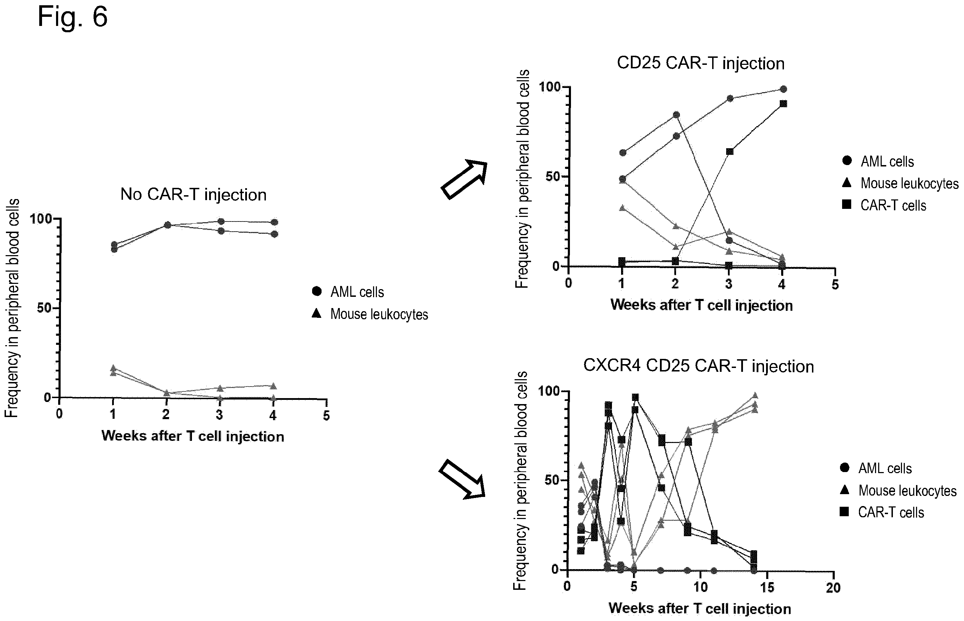

   Fig. 6 shows the frequency (proportion) of AML cells, mouse leukocytes, and CAR-T cells in the peripheral blood cells of PDX mice engrafted with human AML cells. When CAR-T cells were not administered (2 mice), substantially all the cells in the peripheral blood were human AML cells over a period immediately after AML cell transplantation to 4 weeks thereafter. When CD25 CAR-T cells were injected (2 mice), decrease in AML cells accompanied by increase in CAR-T cells were observed in one mouse, but decrease in AML cells was not observed in another mouse. In mice to which CXCR4-expressing CD25 CAR-T cells had been administered (3 mice), in contrast, a significant decrease in AML cells was observed, and AML cell-derived cells were completely eliminated from the circulating blood 3 weeks after CAR-T cell administration. The proportion of CAR-T cells increased while AML cells remained, and gradually decreased. Meanwhile, normal mouse leukocytes increased, and 90% or more cells in the peripheral blood were mouse leukocytes 14 weeks after CAR-T cell administration.
[Figure 7]

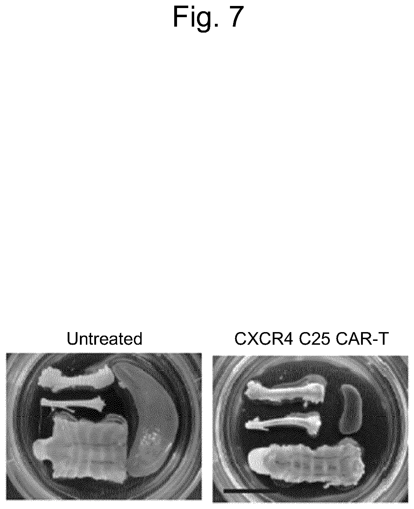

   Fig. 7 shows photographs of the mouse spleen and bone marrow when CXCR4-expressing CD25 CAR-T cells were administered to PDX mice engrafted with human AML cells and when CXCR4-expressing CD25 CAR-T cells were not administered (untreated). While the enlarged spleen and the whitened bone marrow were observed in the untreated mouse, spleen size reduction and the red bone marrow indicating the presence of erythrocytes were observed in a mouse to which CXCR4-expressing CD25 CAR-T cells had been administered. Significant therapeutic effects on AML were confirmed.
[Figure 8]

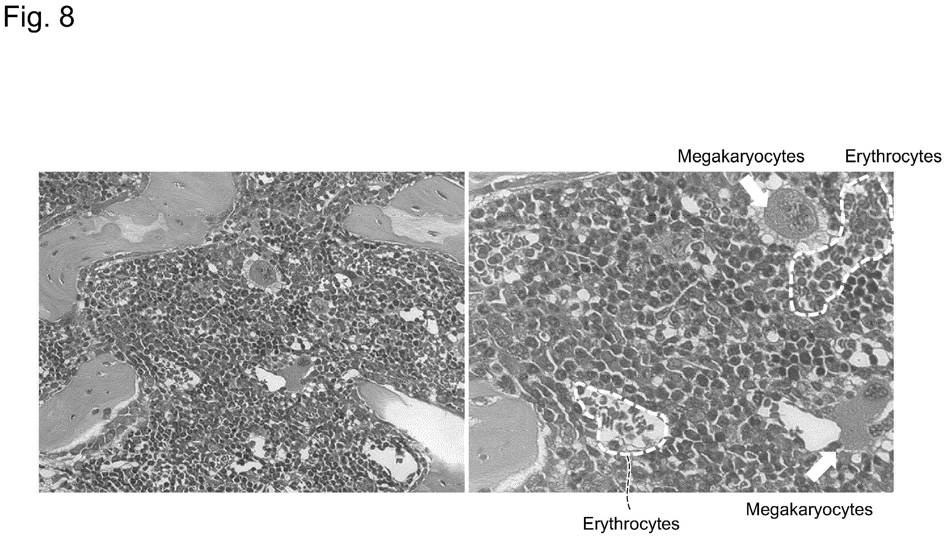

   Fig. 8 shows the results of HE staining of bone marrow tissue of a mouse 4 weeks after CXCR4-expressing CD25 CAR-T cell administration (a PDX mouse engrafted with U390). After AML cell eradication with the aid of CAR-T cells, recovery of normal leucocytes, erythrocytes, and platelet-forming megakaryocytes of mice was confirmed. Left: low-magnification; right: high-magnification.
[Figure 9-1]

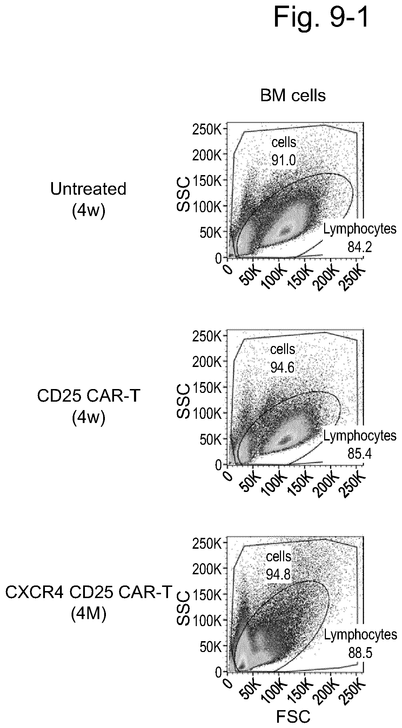

   Fig. 9-1 shows the results of flow cytometric analysis of myeloid cells when CD25 CAR-T cells or CXCR4-expressing CD25 CAR-T cells were administered and when not administered (untreated) to PDX mice engrafted with human AML cells (U390). The results on the untreated sample and on the sample of CD25 CAR-T cell administration were obtained 4 weeks after administration, and the results on the sample of CXCR4-expressing CD25 CAR-T cell administration were obtained 4 months after administration.
[Figure 9-2]

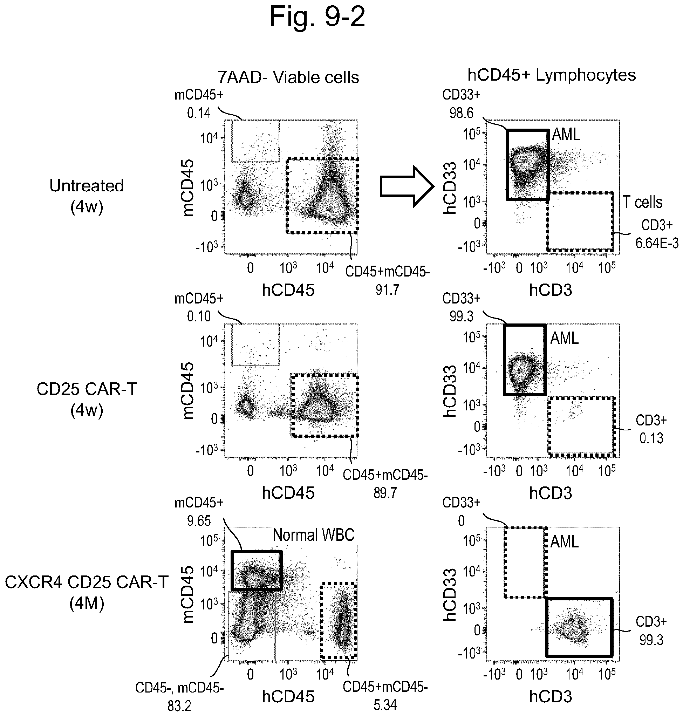

   Fig. 9-2 shows the results of flow cytometric analysis of myeloid cells when CD25 CAR-T cells or CXCR4-expressing CD25 CAR-T cells were administered and when not administered (untreated) to PDX mice engrafted with human AML cells (U390). The results on the untreated sample and on the sample of CD25 CAR-T cell administration were obtained 4 weeks after administration, and the results on the sample of CXCR4-expressing CD25 CAR-T cell administration were obtained 4 months after administration. Only when CXCR4-expressing CD25 CAR-T cells were administered, AML cells completely disappeared, and mouse normal leucocytes (mCD45) were recovered. Complete disappearance of AML cells and the presence of CAR-T cells were maintained for 140 days or longer after injection of CAR-T cells.
[Figure 9-3]

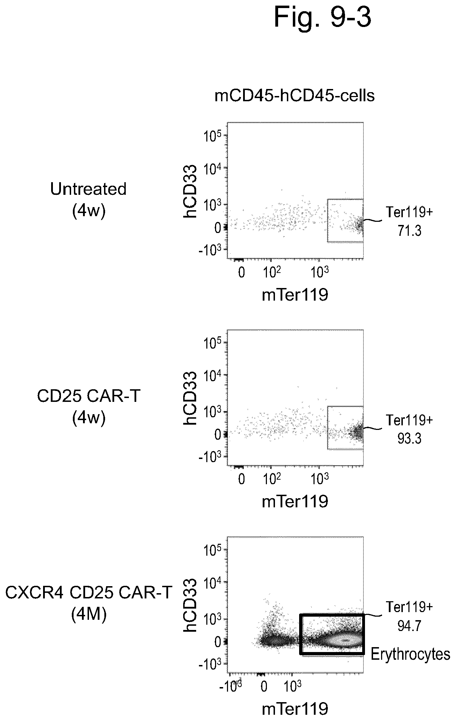

   Fig. 9-3 shows the results of flow cytometric analysis of myeloid cells when CD25 CAR-T cells or CXCR4-expressing CD25 CAR-T cells were administered and when not administered (untreated) to PDX mice engrafted with human AML cells (U390). The results on the untreated sample and on the sample of CD25 CAR-T cell administration were obtained 4 weeks after administration, and the results on the sample of CXCR4-expressing CD25 CAR-T cell administration were obtained 4 months after administration. Only when CXCR4-expressing CD25 CAR-T cells were administered, mouse erythrocytes (Ter119) were recovered.
[Figure 10]

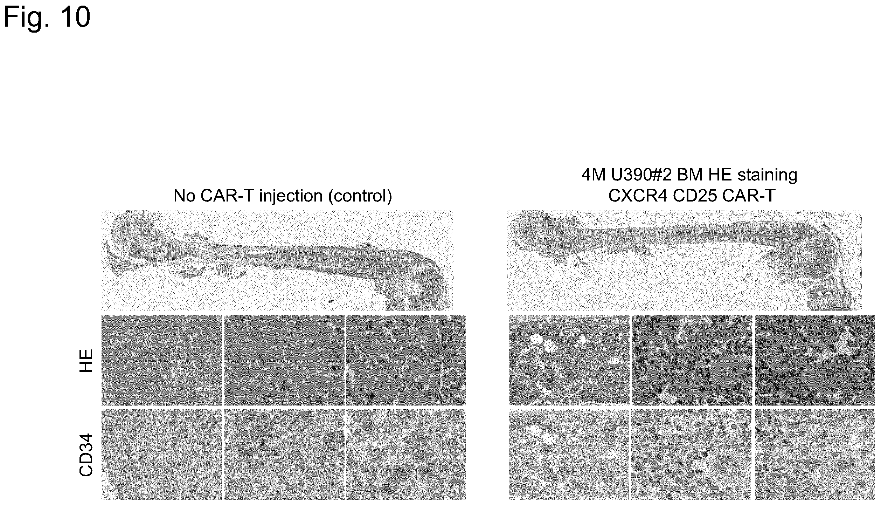

   Fig. 10 shows the results of HE staining of bone marrow tissue or staining of bone marrow tissue with the anti-CD34 monoclonal antibody of AML-engrafted mouse subjected to single administration of CXCR4-expressing CD25 CAR-T cells (right) and a mouse without administration (left), sacrificed 150 days after administration. Effects of AML cell eradication were still maintained 5 months after injection of CAR-T cells.
[Figure 11-1]

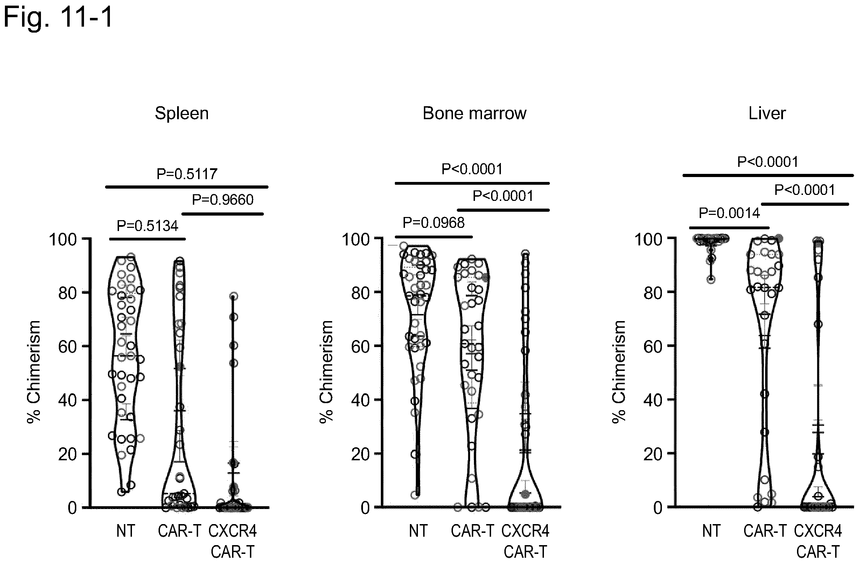

   Fig. 11-1 shows the proportions (% chimerism) of leukemic cells in spleen cells, myeloid cells, and liver cells obtained from PDX mice engrafted with cells derived from 4 AML patients and sacrificed several weeks after administration of CD25 CAR-T or CXCR4-expressing CD25 CAR-T cells. One dot indicates one recipient mouse. In spleen, leukemic cells were killed in a large number of recipient mice when CXCR4-expressing CD25 CAR-T cells were administered. In bone marrow and liver, leukemic cells remained at high frequency in many mice when CD25 CAR-T (CAR-T) cells were administered; however, leukemic cells were killed with a significant probability when CXCR4-expressing CD25 CAR-T cells (CXCR4 CAR-T) were administered. NT: No administration
[Figure 11-2]

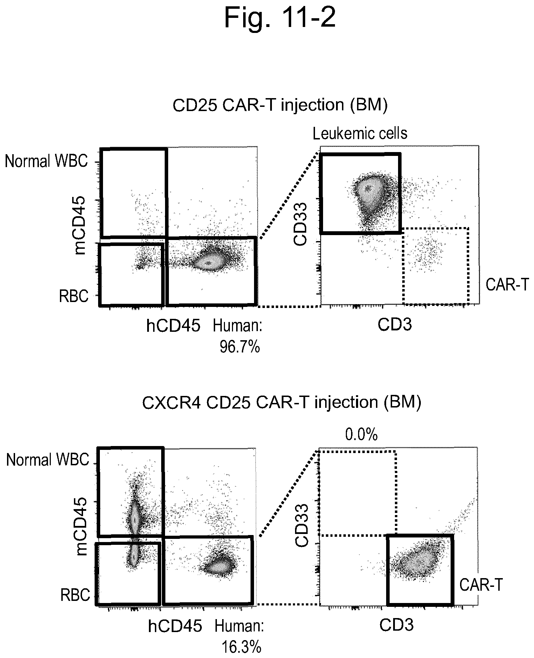

   Fig. 11-2 shows the results of flow cytometric analysis of myeloid cells obtained from PDX mice engrafted with AML-derived cells and sacrificed several weeks after administration of CD25 CAR-T or CXCR4-expressing CD25 CAR-T cells. When CXCR4-expressing CD25 CAR-T cells were administered, leukemic cells disappeared, then the number of CAR-T cells was decreased without being excessively activated, and a large number of normal leucocytes was present in the myeloid cells.
[Figure 12]

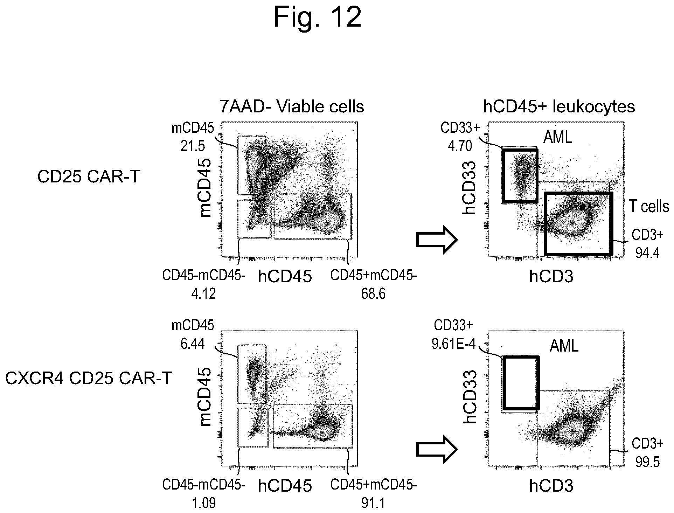

   Fig. 12 shows the results of flow cytometric analysis of liver cells obtained from PDX mice engrafted with human AML cells (U300) and sacrificed 4 weeks after administration of CD25 CAR-T or CXCR4-expressing CD25 CAR-T cells. Also in the liver, increase in T cells and complete disappearance of AML cells were observed when CXCR4-expressing CD25 CAR-T cells had been administered (observed in 4 out of 5 mice), and significantly higher therapeutic effects were observed in comparison with the case when CD25 CAR-T cells had been administered.
[Figure 13-1]

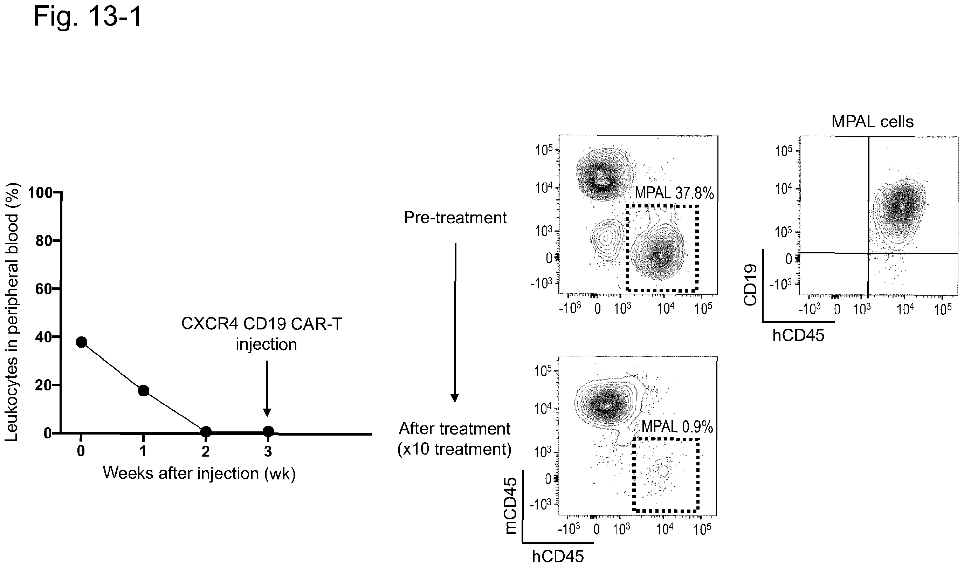

   Fig. 13-1 shows the proportions of leukemic cells in the peripheral blood obtained from PDX mice engrafted with CD19-positive B-cell mixed phenotype acute leukemia (MPAL, B/myeloid, U211) cells subjected to treatment with a small molecule therapeutic agent and then to administration of CXCR4-expressing CD19 CAR-T cells (left). While most leukemic cells seemed to have disappeared from the peripheral blood as a result of administration of a small molecule therapeutic agent, in fact, leukemic cells remained in the bone marrow.
[Figure 13-2]

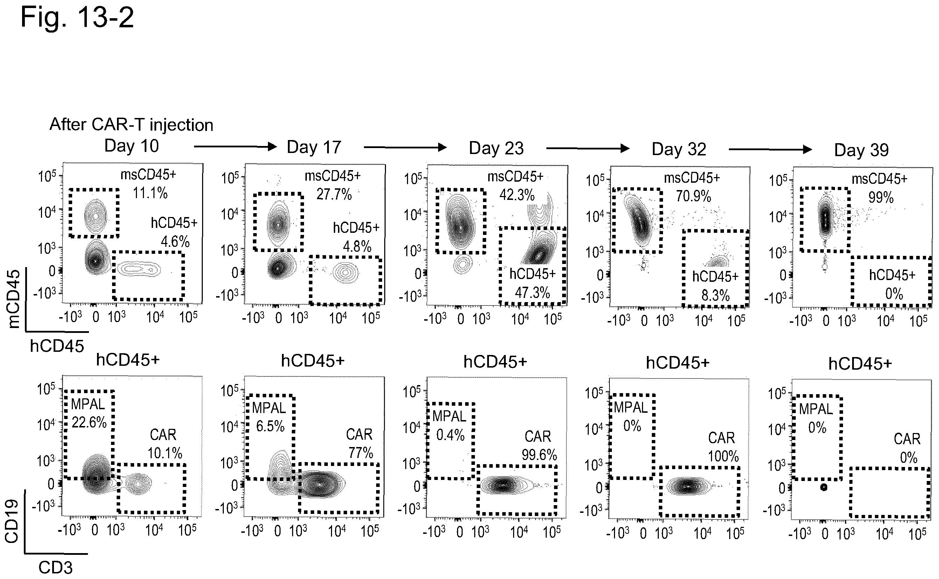

   Fig. 13-2 shows that, upon administration of a small molecule therapeutic agent followed by administration of CXCR4-expressing CD19 CAR-T cells, the proportion of MPAL cells remaining 10 days after administration was reduced to 0.4% 23 days after administration, and MPAL cells completely disappeared 32 days and 39 days after administration.
[Figure 14]

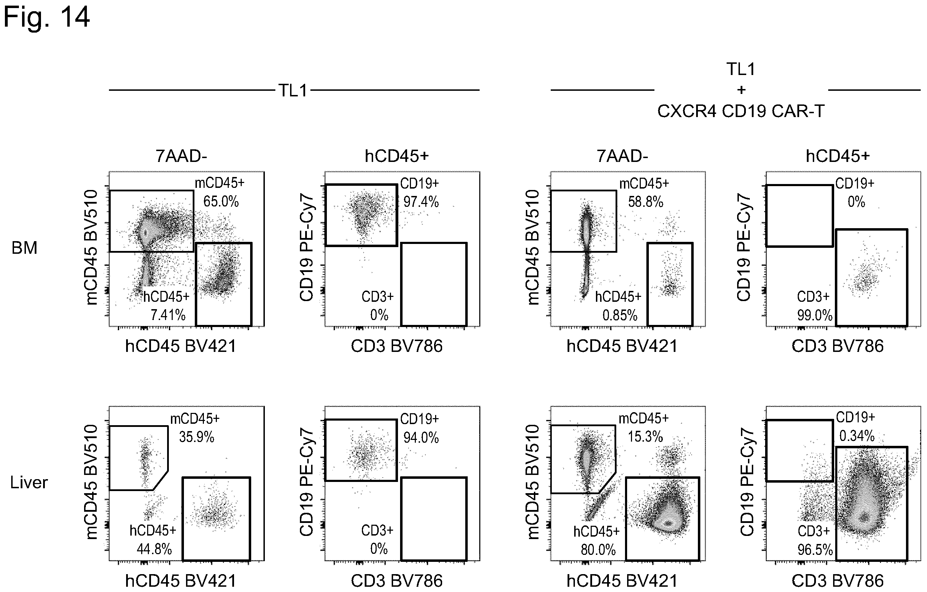

   Fig. 14 shows therapeutic effects of CXCR4-expressing CD19 CAR-T (CXCR4 CD19 CAR-T) on xenograft mouse models using the Burkitt's lymphoma cell line TL1. Charts on the left side show the results of flow cytometry of bone marrow and liver of mice to which CAR-T cells were not administered. The majority of human CD45-positive cells were positive for CD19 as a B cell marker, and a large part of the bone marrow and the liver were accounted for by lymphoma cells. Concerning mice to which CXCR4 CD19 CAR-T had been administered (the right side), the majority of human CD45-positive cells were CAR-T cells (CD3-positive) both in the bone marrow and the liver, and lymphoma cells have substantially disappeared.

### Embodiments of the Invention

An embodiment of the present invention provides a cell co-expressing a chimeric antigen receptor (CAR) protein and a CXCL12 receptor protein on its cell membrane.

### <Chimeric antigen receptor (CAR) protein>

The term "chimeric antigen receptor (CAR)" used herein refers to a modified receptor that can impart its target specificity to a cell. Hereafter, the chimeric antigen receptor or chimeric antigen receptor protein may be referred to as "CAR."

Cells to which target specificity is imparted by CAR are not particularly limited, and a preferable example thereof is T cells. Examples of T cells that can be used include naive T cells, central memory T cells, effector memory T cells, and any combinations thereof. T cells may be inflammatory T lymphocytes, cytotoxic T lymphocytes, or helper T lymphocytes. In particular, cytotoxic T lymphocytes can be used. In an aspect, T cells are CD4+ T lymphocytes or CD8+ T lymphocytes. In a particular aspect, T cells modified according to the present invention are human T cells.

T cells can be obtained from a subject, such as a human patient, by any of a wide variety of non-limited methods before proliferation of the cells according to the present invention and/or gene recombination. T cells can be obtained from a wide variety of non-limited sources, including peripheral blood monocytes, bone marrow, lymph node tissue, cord blood, thymic tissue, tissue derived from the site of infection, ascites fluid, pleural fluid, spleen tissue, and tumors. In an aspect, any number of T cells that are available and known in the art can be used. In another aspect, the cells may be obtained from a healthy donor or a patient diagnosed to have cancer. In another aspect, the cells are some of a mixed population of cells exhibiting different phenotypic features. Cells modified to express chimeric antigen receptors are referred to as "CAR-T cells" herein for the convenience of description.

The structure of the chimeric antigen receptor (CAR) protein is well known in the art, and a common CAR structure can be used in the present invention. Specifically, CAR used in the present invention comprises a target-binding domain binding specifically to a target molecule, a transmembrane domain, and an intracellular signaling domain. The term "domain" used herein refers to a region in a polypeptide, which is folded into a particular structure independently of other regions.

In the present invention, molecules targeted by CAR can be adequately selected from among, for example, antigens expressed on a tumor cell surface, such as differentiation cluster molecules, including CD16, CD64, CD78, CD96, CLL1, CD116, CD117, CD71, CD45, CD71, CD123, and CD138, tumor-associated surface antigens, such as ErbB2 (HER2/neu), carcinoembryonic antigen (CEA), Lewis-Y (TAA-LeY), epithelial cell adhesion molecule (EpCAM), epidermal growth factor receptor (EGFR), EGFR variant III (EGFRvIII), CD19, CD20, CD30, CD40, disialoganglioside GD2, tube epithelial mucin, gp36, TAG-72, sphingoglycolipid, neuroglioma-associated antigen, β-human chorionic gonadotropin, α-fetoprotein (AFP), lectin-reactive AFP, thyroglobulin, RAGE-1, MN-CA IX, human telomerase reverse transcriptase, RU1, RU2 (AS), intestinal carboxylesterase, mut hsp70-2, M-CSF, prostase, prostase-specific antigen (PSA), PAP, NY-ESO-1, LAGA-1a, p53, prostein, PSMA, survivin, and telomerase, prostate carcinoma tumor antigen-1 (PCTA-1), MAGE, ELF2M, neutrophil elastase, ephrin B2, CD22, insulin growth factor (IGF1)-I, IGF-II, IGFI receptor, mesothelin, major histocompatibility complex (MHC) molecule, 5T4, ROR1, Nkp30, NKG2D, tumor interstitial antigen, fibronectin extra domain A (EDA) and extra domain B (EDB), and tenascin-C A1 domain (TnC A1) and fibroblast-associated protein (fap), and cell line-specific antigens or tissue-specific antigens, such as CD3, CD4, CD7, CD8, CD24, CD25 (IL-2 receptor alpha chain), CD32, CD33, CD34, CD123, CD133, CD138, CTLA-4, B7-1(CD80), B7-2(CD86), CD180, GM-CSF, cytokine receptor, endoglin, major histocompatibility complex (MHC) molecule, and lymphoblastic leukemia antigens, such as TNFRSF17(UNIPROT Q02223), SLAMF7, GPRC5D, FKBP11, KAMP3, ITGA8, PRAME, and FCRL5.

In the present invention, molecules targeted by CAR are preferably antigens that are expressed in tumor cells at more significant or apparent levels compared with other cells. Examples thereof include, but are not limited to, CD7, CD19, CD20, GD2, CD22, CD25 (IL-2 receptor alpha chain), CD30, CD33, CD44, CD96, CD123, CD180, CEA, Her2/neu, MUC1, MUC4, MUC6, EGFR, PRAME, VEGFR2, GM-CSFR, IL-11Rα, IL-13α2, and Lewis-Y(TAA-LeY).

In the present invention, molecules targeted by CAR are more preferably antigens that are expressed in particular blood tumor cells at more significant or apparent levels compared with other cells. Examples thereof include, but are not limited to, CD7, CD19, CD25 (IL-2 receptor alpha chain), CD33, CD123, and tumor-associated antigen Lewis-Y (TAA-LeY).

For the treatment and/or the prevention of relapse of acute myeloid leukemia (AML), for example, CD7, CD25, CD96, CD123, PRAME, and CD180 can be targeted. For the treatment and/or the prevention of relapse of B-cell acute lymphatic leukemia (B-ALL) and mixed phenotype acute leukemia (MPAL), for example, CD19, CD20, CD32, and CD180 can be targeted. For the treatment and/or the prevention of relapse of T-cell acute lymphatic leukemia (T-ALL), MPAL, and chronic myeloid leukemia (CML), for example, CD7 can be targeted.

In the present invention, CAR can target a single molecule. In order to target a plurality of molecules, a plurality of CAR-T cells targeting relevant molecules can also be used. In order to target a plurality of molecules, in addition, CARs targeting relevant molecules can also be introduced into the same cell.

In the present invention, it is particularly preferable that CAR target a cell surface antigen selected from among CD25 (IL-2 receptor alpha chain), CD19, and CD7, the expression of which is confirmed in a blood tumor cell.

CD25 (IL-2 receptor alpha chain) has heretofore been reported as a marker for poor prognosis in AML. It has been reported that CD25 is expressed excessively in AML-initiating cells compared with normal D34+CD38- hematopoietic stem/progenitor cells (HSPC) (Saito et al., Science Translational Medicine, 2010).

Gene sequence and other information of CD25 is registered under Gene ID: 3559 in the database of, for example, the National Center for Biotechnology Information (NCBI), and such information can be obtained, according to need.

CD19 is known to be expressed in diffuse large B-cell lymphoma (DLBCL), follicular lymphoma, and B-cell acute lymphatic leukemia (B-ALL) and not expressed in non-hematopoietic cells, myeloid cells, erythrocytes, and T cells. We have confirmed CD19 expression in B cell mixed phenotype acute leukemia (MPAL).

Gene sequence and other information of CD19 is registered under Gene ID: 930 in the database of, for example, NCBI, and such information can be obtained, according to need.

CD7 is a 40 kDa type I transmembrane glycoprotein expressed on a thymocyte and a mature T cell, and it is also used as a marker for T-cell acute lymphatic leukemia (T-ALL). We have confirmed CD7 expression in T cell mixed phenotype acute leukemia (MPAL) and chronic myeloid leukemia (CML).

Gene sequence and other information of CD7 is registered under Gene ID: 924 in the database of, for example, NCBI, and such information can be obtained, according to need.

The target-binding domain of a CAR protein can comprise a single-chain antibody (scFv) fragment comprising a heavy chain (H chain) variable region and a light chain (L chain) variable region of a monoclonal antibody that binds specifically to, for example, the target molecule described above. A person skilled in the art can readily prepare an scFv fragment that can be used for a monoclonal antibody and CAR reacting with a particular antigen. A commercially available scFv fragment can also be used. Examples of target-binding domains that are preferably used in the present invention include scFv fragments binding specifically to CD25, CD19, or CD7.

Examples of target-binding domains that can be used include a ligand that binds specifically to a target molecule, such as an affibody, a ligand-binding domain derived from a naturally-occurring receptor, such as a soluble protein/peptide ligand, of a receptor on a tumor cell, and a peptide.

The CAR protein can optionally comprise an "extracellular spacer domain" between the target-binding domain located extracellularly and the membrane-binding domain. The extracellular spacer domain is preferably a sequence that accelerates binding between CAR and a target molecule and enhances signal transmission into a cell. For example, an Fc fragment of an antibody or a fragment or a derivative thereof, a hinge region of an antibody or a fragment or a derivative thereof, a CH2 region of an antibody, a CH3 region of an antibody, an artificial spacer sequence, or any combination thereof can be used.

The CAR protein comprises an extracellular domain comprising a target-binding domain and, optionally, an extracellular spacer domain, a transmembrane domain, and an intracellular domain comprising an intracellular signaling domain and, optionally, a costimulatory domain.

The "transmembrane domain" is a domain having affinity to a lipid bilayer constituting a cell membrane, while the extracellular domain and the intracellular domain are hydrophilic domains. The transmembrane domain is not particularly limited, provided that the CAR protein can be present on a cell membrane and functions of the target-binding domain and the intracellular signaling domain are not impaired. A polypeptide derived from a protein that is the same as the costimulatory domain described below may serve as a transmembrane domain. For example, a CD28, CD3ε, CD8α, CD3, CD4, or 4-1BB transmembrane domain can be used.

The CAR protein can comprise a "costimulatory domain," according to need. A costimulatory domain binds specifically to a costimulatory ligand, and cell-mediated costimulatory responses, such as CAR-T cell proliferation, cytokine production, function differentiation, and target cell death, are mediated thereby, although costimulatory responses are not limited thereto. Examples of costimulatory domains that can be used include CD27, CD28, 4-1BB (CD137), CD134 (OX40), Dap10, CD27, CD2, CD5, CD30, CD40, PD-1, ICAM-1, LFA-1 (CD11a/CD18), TNFR-1, TNFR-II, Fas, and Lck. For example, human 4-1BB (GenBank:U03397.1) can be used as a costimulatory domain.

The CAR protein comprises an "intracellular signaling domain." The intracellular signaling domain transmits signals that are necessary for immunocytes to exert effector functions. Examples of intracellular signaling domains that can be used include a human CD3ζ chain, FcyRIII, FceRI, a cytoplasmic terminal of an Fc receptor, a cytoplasmic receptor having an immunoreceptor tyrosine activation motif (ITAM), and any combination thereof. For example, a human CD3ζ chain (e.g., NCBI Accession No. NM_000734.3, nucleotides 299-637) can be used as an intracellular signaling domain.

In the first-generation CAR, a signaling domain was derived from CD3ζ or a cytoplasmic region of a Fc receptor γ chain. While the first-generation CAR was found to be capable of directing cytotoxicity of T cells, long-term proliferation *in vivo* and anti-tumor activity thereof were considered insufficient. In order to improve CAR-T cell viability and increase the proliferation thereof, a signaling domain derived from a costimulatory molecule, such as CD28, OX-40 (CD134), and 4-1BB (CD137), is used alone (second-generation), or in combination (third-generation).

Fig. 2 A shows an example of a CAR construct that is preferably used in the present invention. In this example, the target-binding domain is an scFV fragment targeting CD25. In the present invention, the target-binding domain of CAR can be an scFV fragment targeting CD19. In the present invention, the target-binding domain of CAR can be an scFV fragment targeting CD7.

A method for obtaining a monoclonal antibody against a target is known in the art. A person skilled in the art can obtain a monoclonal antibody recognizing a selected target as an antigen, and a commercially available monoclonal antibody can be obtained. A monoclonal antibody or an scFV fragment thereof can be synthesized based on the general technical knowledge in the art, and a gene encoding the same can be synthesized by obtaining the nucleotide sequence thereof.

### <CXCL12 receptor protein>

The cell according to the present invention expresses CAR and the CXCL12 receptor protein on its surface.

The CXC chemokine ligand 12 (CXCL12) is a small molecule protein comprising 89 amino acids that is also referred to as SDF (stromal cell-derived factor)-1, which belongs to the chemokine CXC family. CXCL12 is a strong chemoattractant factor for lymphocytes, which plays a key role in angiogenesis by, for example, inducing endothelial progenitor cells from the bone marrow. CXC motif chemokine receptor 4 (CXCR4, Fusin, CD184) known as a CXCL12 receptor is a 7-transmembrane G protein coupled receptor, which is known to be expressed in neutrophils, monocytes, dendritic cells, NK cells, B cells, T cells, and platelets.

CXCL12/SDF1 is a chemokine ligand that accelerates homing and migration of human and mouse hematopoietic cells or immunocytes to multiple organs including bone marrow and liver. While it is not intended to be bound by any theory, co-expression with the CXCL12 receptor, such as CXCR4, is considered to enable the CAR-T cells to more efficiently home to bone marrow in comparison with CAR-T cells without CXCR4 expression. A CXCL12 receptor that is preferably used in the present invention is CXCR4.

Human CXCR4 is a protein consisting of 360 amino acids encoded by the CXCR4 gene, and the nucleotide sequence of the gene and the amino acid sequence of the protein are registered under Gene ID: 7852 and Accession No: CAA12166 in the database of, for example, the National Center for Biotechnology Information (NCBI), and the gene and the protein can be obtained on the basis of such information.

We have found that the CAR-T cells expressing the CXCL12 receptor protein exert cytotoxic activity against target cells significantly superior to that of the CAR-T cells not expressing the CXCL12 receptor protein. Expression of the CXCL12 receptor protein, such as CXCR4, on a cell may be expression of the entire CXCL12 receptor protein. Alternatively, such expression may be expression of a part of the protein; i.e., a functional fragment, provided that it binds specifically to CXCL12 and improves cytotoxic activity of CAR-T cells.

For example, the CAR-T cells expressing CXCR4 and targeting CD25 (which may be referred to as "CXCR4 CD25 CAR-T" herein) exerted excellent anti-tumor activity against acute myeloid leukemia (AML). Accordingly, the cell according to an aspect of the present invention is a cell that co-expresses CXCR4 and CD25-targeting CAR.

The CAR-T cells expressing CXCR4 and targeting CD19 (which may be referred to as "CXCR4 CD19 CAR-T" herein) exerted excellent anti-tumor activity against CD19-positive acute lymphatic leukemia (ALL), B-cell acute lymphatic leukemia (B-ALL), B-cell mixed phenotype acute leukemia (MPAL), and Burkitt's lymphoma. Accordingly, the cell according to an aspect of the present invention is a cell that co-expresses CXCR4 and CD19-targeting CAR.

At present, ALL is treated with the single use of a small molecule therapeutic agent, such as a BIRC inhibitor (e.g., AZD5582), a BCL-2 inhibitor (e.g., venetoclax), or a steroid drug, or with the use thereof in combination; however, it may not be possible to eradicate ALL only with such small molecule therapeutic agent. We have subjected mice engrafted with human ALL cells to treatment only with a small molecule therapeutic agent, and confirmed that, although leukemic cells seemed to have disappeared from the peripheral blood, in fact, leukemic cells remained in the bone marrow, and that relapse may be induced. In contrast, ALL cells that had increased after the treatment with a small molecule agent (i.e., relapsed ALL) were completely eliminated as a result of administration of CXCR4-expressing CD19 CAR-T cells.

The CAR-T cells expressing CXCR4 and targeting CD7 (which may be referred to as "CXCR4 CD7 CAR-T" herein) exert significant therapeutic effects on CD7-positive T-cell acute lymphatic leukemia (T-ALL), T-cell mixed phenotype acute leukemia (MPAL), and chronic myeloid leukemia (CML). Accordingly, the cell according to an aspect of the present invention is a cell that co-expresses CXCR4 and CD7-targeting CAR.

As described above, the CAR-T cells expressing the CXCL12 receptor protein exert excellent effects of treatment and/or prevention of relapse on tumor cells. Accordingly, the present invention also provides an agent exhibiting anti-tumor activity against tumor cells that comprises the cells according to the present invention.

Target diseases for the treatment and/or the prevention of relapse with the use of the agent according to the present invention are neoplastic diseases, and, in particular, neoplastic diseases of the blood. Examples of neoplastic diseases of the blood include, but are not limited to, leukemia and malignant lymphoma.

Leukemia is classified into myeloid leukemia and lymphocytic leukemia. Myeloid leukemia includes acute myeloid leukemia (AML), acute promyelocytic leukemia, chronic myeloid leukemia (CML), and osteomyelodysplasia syndrome, and lymphocytic leukemia includes acute lymphatic leukemia (ALL) (e.g., B-cell acute lymphatic leukemia (B-ALL), T-cell acute lymphatic leukemia (T-ALL), and mixed phenotype acute leukemia (MPAL)), chronic lymphatic leukemia (CLL), and adult T cell leukemia/lymphoma.

Malignant lymphoma includes Hodgkin's lymphoma (HL) and non-Hodgkin's lymphoma (NHL), and non-Hodgkin's lymphoma is classified into, for example, B-cell lymphoma, T-cell lymphoma, and NK-cell lymphoma. Examples of B-cell malignant lymphoma include diffuse large B-cell lymphoma, Burkitt's lymphoma, chronic lymphatic leukemia/small lymphocytic lymphoma, lymphoplasmacytic lymphoma, splenic marginal zone lymphoma, extranodal marginal zone lymphoma of mucosa-associated lymphoid tissue (MALT lymphoma), nodal marginal zone lymphoma, follicular lymphoma, and mantle cell lymphoma. Examples of T-cell malignant lymphoma include peripheral T cell lymphoma, not otherwise specified, enteropathy-associated T-cell lymphoma, anaplastic large cell lymphoma, hepatosplenic T-cell lymphoma, adult T cell leukemia/lymphoma, extranodal NK/T-cell lymphoma, nasal type, angioimmunoblastic T-cell lymphoma, T-cell large granular lymphocytic leukemia, adult T cell leukemia/lymphoma, mycosis fungoides/Sezary's syndrome, primary cutaneous anaplastic large cell lymphoma, and rapidly progressive NK cell leukemia.

Target diseases for the treatment and/or the prevention of relapse with the use of the agent according to the present invention are, for example, neoplastic diseases selected from the group consisting of acute myeloid leukemia (AML), adult T cell leukemia, B-cell acute lymphatic leukemia (B-ALL), T-cell acute lymphatic leukemia (T-ALL), mixed phenotype acute leukemia (MPAL), chronic myeloid leukemia (CML), Hodgkin's lymphoma, and non-Hodgkin's lymphoma.

The agent according to the present invention is an anticancer agent against the neoplastic diseases mentioned above. The agent according to the present invention; that is, the CAR-T cells, may be used alone. In accordance with various conditions, such as a type of cancer to be treated and progression of symptoms, or at a physician's discretion, the agent according to the present invention may be used in combination with a further anti-tumor agent and/or anti-tumor therapy with different mechanisms.

Further anti-tumor agents are not particularly limited, and examples thereof include antimetabolites, platinum-based drugs, microtubule inhibitors, topoisomerase inhibitors, molecular-targeted agents, and steroid drugs.

Examples of antimetabolites include 5-fluorouracil (5-FU), trifluridine, fludarabine (or an active metabolite; fludarabine nucleoside), cytarabine, gemcitabine, decitabine, guadecitabine, and azacytidine. Examples of platinum-based drugs include cisplatin, oxaliplatin, and carboplatin. Examples of microtubule inhibitors include paclitaxel, docetaxel, vinblastine, vincristine, vindesine, vinorelbine, and eribulin. Examples of topoisomerase inhibitors include irinotecan and etoposide.

Examples of molecular-targeted agents include CSF1R inhibitors, TIE2 inhibitors, TRKB inhibitors, ATR inhibitors, Chk1 inhibitors, HSP90 inhibitors, PARP inhibitors, EGFR inhibitors, Her2 inhibitors, VEGFR inhibitors, PDGFR inhibitors, MET inhibitors, AXL inhibitors, RET inhibitors, FLT3 inhibitors, KIT inhibitors, HCK inhibitors, BIRC inhibitors (e.g., AZD5582), and BCL-2 inhibitors (e.g., venetoclax). As HCK inhibitors and BCL-2 inhibitors, for example, those disclosed in JP 2019-529423 A may be used.

Examples of steroid drugs include dexamethasone.

Examples of other anti-tumor therapy include surgery and radiotherapy.

The agent according to the present invention is found to be able to exert more potent therapeutic effects against leukemia when used in combination with a BIRC inhibitor and/or a BCL-2 inhibitor, although use thereof is not limited thereto. Accordingly, an aspect of the present invention concerns combination therapy including an agent containing CAR-T cells that express a CXCL12 receptor protein and a BIRC inhibitor and/or a BCL-2 inhibitor. For example, the cells co-expressing a chimeric antigen receptor (CAR) protein and a CXCL12 receptor protein on the cell membrane according to the present invention can be administered in combination with a BIRC inhibitor (e.g., AZD5582) to a patient with hematologic malignancies. Also, the cells co-expressing a chimeric antigen receptor (CAR) protein and a CXCL12 receptor protein on the cell membrane according to the present invention can be administered in combination with a BCL-2 inhibitor (e.g., venetoclax) to a patient with hematologic malignancies.

Administration of the agent containing CAR-T cells that express a CXCL12 receptor protein according to the present invention and administration of the further anti-tumor agent and/or anti-tumor therapy can be performed simultaneously, continuously, or separately. In an aspect, the further anti-tumor agent can be administered and/or anti-tumor therapy can be performed simultaneously with the administration of the agent according to the present invention. In an aspect, the further anti-tumor agent can be administered and/or anti-tumor therapy can be performed after the administration of the agent according to the present invention. In an aspect, the further anti-tumor agent can be administered and/or anti-tumor therapy can be performed before the administration of the agent according to the present invention. In an aspect, the further anti-tumor agent can be administered and/or anti-tumor therapy can be performed simultaneously with the administration of the agent according to the present invention. In another aspect, the agent according to the present invention can be administered in the case of relapse after the treatment via administration of the anti-tumor agent and/or the anti-tumor therapy.

The present invention also provides a pharmaceutical composition comprising the agent according to the present invention and a pharmaceutically acceptable carrier. The pharmaceutical composition can contain, as an active ingredient, the agent according to the present invention alone or in combination with other active ingredients. The pharmaceutical composition according to the present inventio is intended for the treatment and/or the prevention of relapse of the neoplastic diseases mentioned above. For example, the pharmaceutical composition according to the present invention can be used for the treatment and/or the prevention of relapse of neoplastic diseases selected from the group consisting of acute myeloid leukemia (AML), adult T cell leukemia, B-cell acute lymphatic leukemia (B-ALL), T-cell acute lymphatic leukemia (T-ALL), mixed phenotype acute leukemia (MPAL), chronic myeloid leukemia (CML), Hodgkin's lymphoma, and non-Hodgkin's lymphoma.

The term "pharmaceutically acceptable carrier" used herein refers to a nontoxic carrier that is necessarily or preferably added for preparation and administration of the pharmaceutical composition without impairing the activity of the active ingredients. For example, an aqueous medium, such as water or physiological saline, an excipient, an isotonizing agent, a buffer, a stabilizer, or a gelling agent can be adequately added.

The agent or the pharmaceutical composition according to the present invention can be administered topically or systemically. While the route of administration is not limited, intravenous administration is preferable for the treatment of, for example, leukemia. A dose of the agent according to the present invention would vary in accordance with body weight, age, and severity of disease of the subject. While a dose is not particularly limited, for example, the cell is administered in the range of 10⁴ to 10¹⁰ or 10⁴ to 10⁹ cells per kg body weight of the target. The agent or the pharmaceutical composition according to the present invention can be administered once. Alternatively, the agent or the pharmaceutical composition according to the present invention can be administered a plurality of times, for example, 1 to several times a day, every 2 days, every 3 days, every week, every 2 weeks, every month, every 2 months, or every 3 months.

### <Method for preparing cells>

The present invention also provides a method for preparing the cells according to the present invention comprising introducing a first polynucleotide encoding a chimeric antigen receptor (CAR) protein and a second polynucleotide encoding a CXCL12 receptor protein into cells.

A polynucleotide of interest can be readily prepared in accordance with a conventional technique. Concerning the polynucleotide encoding the chimeric antigen receptor (CAR) protein, nucleotide sequences encoding the respective amino acid sequences of domains (a target-binding domain, an extracellular spacer domain, a transmembrane domain, a costimulatory domain, and an intracellular signaling domain) can be obtained based on NCBI RefSeq IDs or GenBank Accession numbers indicating the amino acid sequences thereof, and the first polynucleotide of the present invention can be prepared by ligating the polynucleotides encoding the respective domains in accordance with standard molecular biological and/or chemical procedures. For example, nucleic acids can be synthesized on the basis of these nucleotide sequences. Also, DNA fragments obtained by polymerase chain reaction (PCR) from a cDNA library can be combined to prepare the polynucleotide of the present invention.

Concerning the second polynucleotide encoding the CXCL12 receptor protein, nucleotide sequences encoding the respective amino acid sequences can be obtained based on NCBI RefSeq IDs or GenBank Accession numbers indicating the amino acid sequences thereof, and the second polynucleotide of the present invention can be prepared in accordance with standard molecular biological and/or chemical procedures.

The first polynucleotide and the second polynucleotide can be introduced into cells, such as T cells, in accordance with any adequate method known in the art. Examples of adequate methods for introducing nucleic acid molecules into cells include, but are not limited to, stable transformation by which polynucleotides are incorporated into the cellular genome, transient transformation by which polynucleotides are not incorporated into the cellular genome, and a virus-mediated method. For example, polynucleotides may be introduced into cells with the aid of a recombinant viral vector (e.g., retroviral, lentiviral, or adenoviral vector) or liposome. Examples of transient transformation techniques include microinjection, electroporation, and particle bombardment.

A polynucleotide to be introduced into a cell may be DNA or RNA. In an aspect, a nucleic acid molecule to be introduced is DNA. In an aspect, a nucleic acid molecule to be introduced into a cell is RNA, and, in particular, mRNA encoding a CAR protein or a CXCL12 receptor protein.

For example, the chimeric antigen receptor (CAR) protein can target a cell surface antigen selected from among CD25 (IL-2 receptor alpha chain), CD19, and CD7.

The first polynucleotide and the second polynucleotide can be introduced into a cell using a single vector. Alternatively, the first polynucleotide and the second polynucleotide can be introduced into a cell using different vectors.

### <Method for treatment>

The present invention further provides a method for treatment of tumors, which comprises administering a therapeutically effective amount of the agent or the pharmaceutical composition according to the present invention to a patient. A therapeutically effective amount and an administration regimen can be adequately determined in consideration of, for example, the type of the target disease, progression thereof, and age of the patient. The subjects (patients) to be treated by the method according to the present invention are mammals, such as mice, rats, dogs, cats, rabbits, cattle, horses, sheep, goats, monkeys, and humans. The subjects to be treated by the method according to the present invention are preferably human patients. The subjects can also be non-human mammals engrafted with human tumor cells or non-human animals with neoplastic diseases.

The method according to the present invention is a method for the treatment and/or the prevention of relapse of neoplastic diseases of a subject, which comprises a step of administering cells co-expressing the chimeric antigen receptor (CAR) protein targeting CD25 (IL-2 receptor alpha chain) and the CXCL12 receptor protein on the cell membrane to the subject.

The chimeric antigen receptor (CAR) protein is not limited, and, for example, it can target a cell surface antigen selected from among CD25 (IL-2 receptor alpha chain), CD19, and CD7.

Target neoplastic diseases of the treatment and/or the prevention of relapse in the method according to the present invention may be any of the neoplastic diseases mentioned above. For example, the target disease can be any disease selected from the group consisting of acute myeloid leukemia (AML), adult T cell leukemia, B-cell acute lymphatic leukemia (B-ALL), T-cell acute lymphatic leukemia (T-ALL), mixed phenotype acute leukemia (MPAL), chronic myeloid leukemia (CML), Hodgkin's lymphoma, and non-Hodgkin's lymphoma.

In the method according to the present invention, the cells can be administered once to a subject. In the method according to the present invention, alternatively, the cells can be administered a plurality of times to a subject. In the method according to the present invention, as described above, administration of the agent or the pharmaceutical composition according to the present invention can be performed in combination with administration of a further therapeutic agent, such as a further anti-tumor agent and/or anti-tumor therapy.

In the method according to the present invention, cells may be administered in the range of 10⁴ to 10⁹ cells per kg body weight of the subject once or a plurality of times.

The method according to the present invention can further comprise a step of measuring the expression level of a target cell surface antigen in a tumor cell of the subject before the step of administering the cell. In an aspect, for example, the method according to the present invention can comprise a step of measuring the CD25 expression level in a target tumor cell before the step of administering CXCR4-expressing CD25 CAR-T cells. In another aspect, the method according to the present invention can comprise a step of measuring the CD 19 expression level in a target tumor cell before the step of administering CXCR4-expressing CD19 CAR-T cells. In a further aspect, the method according to the present invention can comprise a step of measuring the CD7 expression level in a target tumor cell before the step of administering CXCR4-expressing CD7 CAR-T cells.

The method according to the present invention can further comprise a step of evaluating therapeutic effects after the step of administering the CXCR4-expressing CAR-T cells. The therapeutic effects can be evaluated based on one or more indices selected from (i) decrease in tumor cells in the blood, (ii) decrease in tumor cells in the bone marrow, (iii) decrease in tumor cells in the spleen, and (iv) suppression of tumor cell infiltration into the liver, in the subject.

### <Use in treatment and/or prevention of relapse of cancer>

The present invention also provides cells co-expressing a chimeric antigen receptor (CAR) protein and a CXCL12 receptor protein on the cell membrane for use in the treatment and/or the prevention of relapse of cancer.

For example, the chimeric antigen receptor (CAR) protein expressed on the cell surface targets a cell surface antigen selected from among CD25 (IL-2 receptor alpha chain), CD19, and CD7.

The cancer may be any disease selected from among the neoplastic diseases mentioned above. For example, the cancer is a neoplastic disease selected from the group consisting of acute myeloid leukemia (AML), adult T cell leukemia, B-cell acute lymphatic leukemia (B-ALL), T-cell acute lymphatic leukemia (T-ALL), mixed phenotype acute leukemia (MPAL), chronic myeloid leukemia (CML), Hodgkin's lymphoma, and non-Hodgkin's lymphoma. The cells may be those mentioned above, and are preferably T cells.

Examples

Hereafter, the present invention is described in greater detail with reference to the examples, although the present invention is not limited to the following examples.

Lentiviral vectors were constructed in the manner described below.

With the use of the lentiviral vector pHR_SFFV (gifted from Dr. Wendell Lim (Addgene plasmid # 79121)), CD25-CAR (pHR_SFFV CD25CAR), mouse CXCR4 (pHR_SFFV mCXCR4), packaging plasmids (pCMVR, pL2, pMD2.G-VSV-G, pAdV (Promega)) (Cho et al 2018 Cell), and the JetPEI transfection reagent (Polyplus), 293T cells were transfected. Thus, transgenes were packaged into lentiviral vectors.

Four days after transfection, the viral supernatant was harvested and centrifuged using Vivaspin 200 (Merck). The concentrated viral supernatant was centrifuged overnight, the supernatant was discarded, and the pellet was lysed using the medium for T cell culture.

Culture and lentiviral infection of primary human T cells were performed in the manner described below.

Human samples were collected with written informed consent from AML patients. Cord blood samples of healthy donors were obtained from the Chubu Cord Blood Bank (Aichi, Japan).

Bone marrow monocytes of AML patients and cord blood monocytes were isolated via density gradient centrifugation. CD34⁺ cells and CD34⁻ cells were separated by autoMACS (Miltenyi) using anti-human CD34 immunomagnetic beads. T cells were enriched using the autoMACS with Pan T cells isolation kit (Miltenyi) from the CD34⁻ population. After T cell isolation, culture was performed in X-Vivo15 (Lonza), 5% fetal bovine serum, 10 mM N-acetyl-L-cysteine (Sigma-Aldrich#A9165), and 55 mM 2-mercaptoethanol (Jang hwan Cho, 2018, Cell).

T cells were stimulated with 25 µl Human T-activator CD3/CD28 DynaBeades (Thermo Scientific #11132D) and adjusted to 1 × 10⁶ cells in 1 ml culture medium. On the following day, T cells were seeded at 1 × 10⁵ cells /100 µl into a well of a 96-well plate for lentiviral infection. CAR lentiviruses (16 to 100 µl) were added to 100 µl of a T cell suspension using vectofusin-1 (10 µg/ml, Miltenyi) and then cultured for 1 day. A half of the medium containing infected T cells was removed, and the same amount of a fresh medium was added. Two days after infection, the infected T cells were analyzed concerning the expression of 25CAR using a CD25-FC fusion protein and concerning the expression of mCXCR4 using an anti-mCXCR4 antibody. After surface protein expression was confirmed, CD25 CAR-T cells were injected into AML mouse models through the retroorbital sinus.

Flow cytometric analysis was performed by labeling the cells with monoclonal antibodies (CD45, CD3, CD4, CD8, CD25, CD33, and mouse CD45) using FACSAria III or FACSCanto II (BD Biosciences).

### [Reference Example 1: Expression of CD25/IL2RA on normal and malignant human hematopoietic cell surfaces]

CD25 (IL-2Rα chain) was identified as a gene expressed differently on AML-initiating cells and on normal CD34⁺CD38⁻ hematopoietic stem/progenitor cells (Saito et al., 2010, Sci. Trans. Med.). In addition, expression of CD25 in AML has been reported as a poor prognostic factor (Gonen et al., 2012, Blood; Nguyen et al., Cancer Research, 2020). AML with diverse genetic abnormalities may result in different CD25 expression levels among cases.

In order to examine heterogeneity of CD25 expression in AML patient's cells, cell surface expression of CD25 in CD33+ leukemic blasts derived from 84 AML cases was analyzed by flow cytometry.

The results demonstrate that 57 cases were CD25-negative, 14 cases showed medium-level expression of CD25, and 13 cases showed high-level expression of CD25. Among the 84 cases, CD25 expression was observed at various levels in AML cells derived from 27 patients (32.2%) (Fig. 1 A and Fig. 1 B).

We also analyzed CD25 expression in immature and mature human hematopoietic cells. As a result, the frequency of CD25-expressing cells was found to be low in hematopoietic stem/progenitor cells (CD34⁺CD38⁻) and myeloid-erythroid progenitor cells (CD34⁺CD38⁺) (Fig. 1 C), which was consistent with our previous report (Saito, et al., 2010, Sci. Trans. Med.). It was also found that less than 5% of monocytes (CD14⁺), granulocytes (CD15⁺), and NK cells (CD56⁺) expressed CD25 protein, and 8.8% of human T cells (CD3⁺) expressed CD25 (Fig. 1 C).

### [Reference Example 2: Cytotoxic activity of CD25-targeting CAR-T cells]

In order to prepare CAR-T cells targeting the CD25 antigen, a lentiviral vector harboring the anti-IL2RA/CD25 protein single-chain variable fragment (scFv) and the intracellular CD3z and CD137 (4-1BB) signaling domains was designed (Fig. 2 A) (Michael C. Milone, et al., 2009, Mol. Therapy, 17 (8): 1453-64).

Subsequently, effects of CAR-T cells for *in vitro* killing on leukemic cells of patients exhibiting various degrees of CD25 expression were evaluated. To this end, *in vitro* T cell-mediated cytotoxicity assays were constituted using AML samples from patients with or without CD25 expression.

As a result of culture with CD25 CAR-T cells for 3 days, viability of CD25-positive primary AML cells (U390, U346) was significantly impaired, and the absolute number of the AML cells was reduced, although such changes were not observed in the case of non-specific activated T cells (U328) (Fig. 2B). AML cell death was not occur with the use of CD25-negative AML cells as a target or CAR-untransduced activated T cells as effectors, indicating that AML cell death was occurred through recognition of CD25 by CAR-T cells.

### [Reference Example 3: CD25 CAR-T cannot eradicate CD25-positive AML in vivo]

Since antigen-specific cytotoxicity of CAR-T cells against CD25-expressing AML of patients was observed *in vitro,* therapeutic effects of the CAR-T cells *in vivo* were evaluated.

NSG mice engrafted with AML cells derived from patients were prepared in the manner described below. NOD.Cg-Prkdc^{scid}Il2rg^{tmlWjl/Sz} (NOD-SCID -IL2rg^{null}) mice developed at the Jackson Laboratory by backcrossing to have a complete null mutation at the Il2rg locus in the NOD.Cg-Prkdc^{scid} (NOD-SCID) strain (Shultz et al., 2005, .J Immunol.) were obtained.

Newborn NOD-SCID-IL2rg^{null} mice were subjected to total body irradiation (150 cGy) and then to intravenous injection of human AML cells. To generate AML-engrafted recipients, 10³ to 10⁵ AML engrafting cells were injected into each recipient (Ishikawa et al., 2007, Nature Biotechnol.). To generate normal human HSC-engrafted recipients, 10⁴ 7AAD lineage (hCD3/hCD4/hCD8)⁻hCD34⁺hCD38⁻ CB cells were injected into each recipient (Ishikawa et al., 2005, Blood). Human peripheral blood cell engraftment was evaluated by retroorbital phlebotomy.

After the presence of AML cells in the peripheral blood of the NSG mice engrafted with AML cells was confirmed, 5 × 10⁶ CD25 CAR-T cells were administered once, the peripheral blood samples were collected every week for 4 weeks, and analyzed by flow cytometry. As a result, as shown in Fig. 3, the proportion of CAR-T cells (CD3+) was increased and the proportion of AML (hCD45+CD33+CD25+) was decreased in human CD45 of all mice, but hCD45⁺hCD33⁺AML blasts remained even 4 weeks after administration. Also, variations in effects were observed, for example, potent effects were observed in Mouse #2, but weak effects were observed in Mouse #1 and Mouse #3.

Mice were subjected to dissection and HE staining of the bone marrow 4 weeks after administration of CD25 CAR-T cells. As a result, as shown in Fig. 4, various types of cells, such as granulocytes and myelocytes, were observed in the bone marrow of immunodeficient mice (normal NSG), the majority of myeloid cells was AML cells in the bone marrow of AML PDX mice even after administration of CD25 CAR-T cells, and there were very few cells of the erythroid lineage, megakaryocytes, and normal leucocytes.

### [Example 1: Effects of CXCR4-expressing CD25 CAR-T cells 1]

Therapeutic effects of the CD25 CAR-T cells in the bone marrow were not optimal. Thus, a CAR construct that would enable the CAR-T cells to more efficiently home to the bone marrow was designed. In order to prepare CXCR4-expressing CD25 CAR-T, both of the pHR_SFFV_mCXCR4 vector and the pHR_SFFV_CD25CAR vector were transfected to 293T cells.

After the CD25 CAR-T cells or the CXCR4-expressing CD25 CAR-T cells were administered to the PDX mice engrafted with AML cells, the peripheral blood samples were collected every week, the cells were separated by flow cytometry, and the proportions of AML cells, mouse leukocytes, and CAR-T cells were inspected. As a result, stable reduction of human AML cells over time during the 4-week observation period and complete elimination of human AML cells 4 weeks after injection were observed (Fig. 5-1 to Fig. 5-3, Fig. 6).

As a result of treatment with the CXCR4-expressing CD25 CAR-T cells, CD33-positive leukemic cells were completely eliminated within 4 weeks, and the effects thereof were maintained for 4 months or longer. After the leukemic cells were reduced, the CAR-T cells were also reduced, and a majority of the peripheral blood was accounted for by mouse CD45-positive cells. Specifically, cells co-expressing CXCR4 were found to exert higher effects in *in vivo* eradication of human AML cells by CD25 CAR-T cells.

### [Example 2: Effects of CXCR4-expressing CD25 CAR-T cells 2]

The spleen and the bone marrow were removed from the PDX mice to which CXCR4-expressing CD25 CAR-T cells had been administered in Example 1, and visually compared with the case without administration. As a result, as shown in Fig. 7, in the mice to which CXCR4-expressing CD25 CAR-T cells had been administered, the reduction of spleen size to a normal size and the red bone marrow indicating recovery of cells of the erythroid lineage were observed.

Fig. 8 shows the results of HE staining of the bone marrow tissue of the mice (U390-engrafted PDX mice) 4 weeks after administration of CXCR4-expressing CD25 CAR-T cells. After AML cells were eradicated with the aid of the CAR-T cells, all of normal leucocytes, erythrocytes, and platelet-forming megakaryocytes of mice were found to have been recovered.

As a result of flow cytometric analysis of myeloid cells, complete disappearance of leukemic cells and recovery of normal cells, such as mouse erythrocytes (Ter119) and leucocytes (mCD45), were observed only when the CXCR4-expressing CD25 CAR-T cells had been administered (Fig. 9-1 to Fig. 9-3), consistent with the results described above.

The effects of the CXCR4-expressing CD25 CAR-T cells on the eradication of AML cells in the bone marrow were maintained even 5 months after injection of the CAR-T cells (Fig. 10).

Also in the liver, increase in T cells and complete disappearance of leukemic cells were observed only when CXCR4-expressing CD25 CAR-T cells had been administered, and significantly higher therapeutic effects, in comparison with the case when CD25 CAR-T cells had been administered, were confirmed (Fig. 11-1 to Fig. 11-2, Fig. 12).

As a result of histological examination, in addition, GVHD or other pathological inflammation was not observed in the liver, the intestine, and the skin of the mice to which CXCR4-expressing CD25 CAR-T cells had been administered (data not shown). In further two cases, the death of human AML cells at a significant level was observed with the CXCR4-expressing CD25 CAR-T cells. In 2 out of the 3 cases examined, infiltration of AML cells in the recipients' liver tissues was observed. Since the liver is an organ which shows strong expression of CXCL-12/SDF1, it is considered that co-expression of CXCR4 in the CD25 CAR-T cells exerted potent therapeutic effects against human AML cells infiltrated into the liver tissues of recipient animals.

As described above, single injection of 5 × 10⁶ CXCR4-expressing CD25 CAR-T cells can be sufficient to achieve long-term treatment responses to CD25-expressing aggressive AML.

### [Example 3: Effects of CXCR4-expressing CD19 CAR-T cells 1]

PDX mice engrafted with CD19-positive B-cell mixed phenotype acute leukemia (MPAL, B/myeloid, U211) cells were subjected to treatment with a BIRC inhibitor (AZD5582) (0.5 mg/kg/day, intraperitoneal administration), a BCL-2 inhibitor (venetoclax) (30 mg/kg/day, oral administration), and dexamethasone (DEX) (30 mg/kg/day, intraperitoneal administration). As shown in Fig. 13-1, the number of leukemic cells (MPAL) in the peripheral blood after administration of 10 times was reduced compared with that before treatment (37.8% to 0.9%), but leukemic cells still remained therein. Leukemic cells were detected not only in the peripheral blood but also in the bone marrow (data not shown).

Since the presence of residual leucocytes may induce relapse, CXCR4-expressing CD 19 CAR-T cells (5 × 10⁶ cells) were administered after treatment with molecular-targeted agents.

As a result, as shown in Fig. 13-2, MPAL cells indicating relapse were present 10 days after administration of CAR-T cells; however, the proportion thereof was reduced to 0.4% 23 days after administration, and the MPAL cells were completely eliminated 32 days and 39 days after administration.

### [Example 4: Effects of CXCR4-expressing CD19 CAR-T cells 2]

Burkitt's lymphoma, which is high-grade B-cell lymphoma, has been treated by chemotherapy with multiple drugs. In the treatment-resistant cases and the relapse-inducing cases, the five-year survival rate is reported to be 50% or lower even when high-dose chemotherapy in combination with autologous hematopoietic stem cell transplantation or homologous hematopoietic stem cell transplantation is performed. That is, such cases have poor prognosis (J. Oncol. Pract., Nov 2018, 14 (11): 665-671).

In this example, xenograft Burkitt's lymphoma mouse models were prepared in the same manner as in Example 1 with the use of the Burkitt's lymphoma cell line TL1 (obtained from Cell Resource Center for Biomedical Research, Institute of Development, Aging, and Cancer, Tohoku University), and effects of administration of 5 × 10⁶ CXCR4-expressing CAR-T cells having binding specificity to CD19 (CXCR4-expressing CD19 CAR-T) were examined.

The diagrams on the left side of Fig. 14 show the results of flow cytometry of the bone marrow (BM) and the liver of the mice without CAR-T cell administration. The majority of human CD45-positive cells were positive for CD19 as a B cell marker, and a large part of the bone marrow and that of the liver were accounted for by lymphoma cells.

In contrast, in the mice to which CXCR4-expressing CD19 CAR-T cells had been administered (the right side), the majority of human CD45-positive cells were CAR-T cells (CD3-positive) both in the bone marrow and the liver, and lymphoma cells have substantially disappeared.

### Industrial Applicability

The present invention can provide a very effective method for the treatment and/or the prevention of relapse of poor-prognosis neoplastic diseases that were difficult to completely cure.

All publications, patents, and patent applications cited herein are incorporated herein by reference in their entirety.

## Claims

1. A cell co-expressing a chimeric antigen receptor (CAR) protein and a CXCL12 receptor protein on the cell membrane.

2. The cell according to claim 1, wherein the CXCL12 receptor is CXCR4.

3. The cell according to claim 1 or 2, wherein the chimeric antigen receptor (CAR) protein targets a cell surface antigen selected from among CD25 (IL-2 receptor alpha chain), CD19, and CD7.

4. The cell according to any one of claims 1 to 3, which is a T cell.

5. An agent exhibiting anti-tumor activity, which comprises the cell according to any one of claims 1 to 4.

6. The agent according to claim 5, which is used in combination with a further anti-tumor agent and/or anti-tumor therapy.

7. A pharmaceutical composition comprising the agent according to claim 5 and a pharmaceutically acceptable carrier.

8. The agent according to claim 5 or 6 or the pharmaceutical composition according to claim 7, for the treatment and/or the prevention of relapse of a neoplastic disease selected from the group consisting of acute myeloid leukemia (AML), adult T cell leukemia, B-cell acute lymphatic leukemia (B-ALL), T-cell acute lymphatic leukemia (T-ALL), mixed phenotype acute leukemia (MPAL), chronic myeloid leukemia (CML), Hodgkin's lymphoma, and non-Hodgkin's lymphoma.

9. A method for preparing the cell according to any one of claims 1 to 4, which comprises introducing a first polynucleotide encoding a chimeric antigen receptor (CAR) protein and a second polynucleotide encoding a CXCL12 receptor protein into a cell.

10. The method according to claim 9, wherein the chimeric antigen receptor (CAR) protein targets a cell surface antigen selected from among CD25 (IL-2 receptor alpha chain), CD19, and CD7.

11. The method according to claim 9 or 10, wherein the first polynucleotide and the second polynucleotide are introduced into a cell by the same vector or separate vectors.

12. A method for the treatment and/or the prevention of relapse of a neoplastic disease in a subject, which comprises a step of administering a cell co-expressing a chimeric antigen receptor (CAR) protein targeting CD25 (IL-2 receptor alpha chain) and a CXCL12 receptor protein on the cell membrane to the subject.

13. The method according to claim 12, wherein the chimeric antigen receptor (CAR) protein targets a cell surface antigen selected from among CD25 (IL-2 receptor alpha chain), CD19, and CD7.

14. The method according to claim 12 or 13, wherein the neoplastic disease is selected from the group consisting of acute myeloid leukemia (AML), adult T cell leukemia, B-cell acute lymphatic leukemia (B-ALL), T-cell acute lymphatic leukemia (T-ALL), mixed phenotype acute leukemia (MPAL), chronic myeloid leukemia (CML), Hodgkin's lymphoma, and non-Hodgkin's lymphoma.

15. The method according to any one of claims 12 to 14, wherein the cell is administered once to a subject.

16. The method according to any one of claims 12 to 15, wherein the cell is administered in the range of 10⁴ to 10⁹ cells per kg body weight of the subject.

17. The method according to any one of claims 12 to 16, which further comprises a step of measuring the expression level of a target cell surface antigen in a tumor cell of the subject before the step of administering the cell.

18. The method according to any one of claims 12 to 17, which further comprises a step of evaluating therapeutic effects after the step of administering the cell.

19. The method according to claim 18, wherein the therapeutic effect is evaluated based on one or more indices selected from (i) decrease in tumor cells in the blood, (ii) decrease in tumor cells in the bone marrow, (iii) decrease in tumor cells in the spleen, and (iv) suppression of tumor cell infiltration into the liver, in the subject.

20. A cell for use in the treatment and/or the prevention of relapse of a cancer, which co-expresses a chimeric antigen receptor (CAR) protein and a CXCL12 receptor protein on the cell membrane.

21. The cell according to claim 20, wherein the chimeric antigen receptor (CAR) protein targets a cell surface antigen selected from among CD25 (IL-2 receptor alpha chain), CD19, and CD7.

22. The cell according to claim 20 or 21, wherein the cancer is a neoplastic disease selected from the group consisting of acute myeloid leukemia (AML), adult T cell leukemia, B-cell acute lymphatic leukemia (B-ALL), T-cell acute lymphatic leukemia (T-ALL), mixed phenotype acute leukemia (MPAL), chronic myeloid leukemia (CML), Hodgkin's lymphoma, and non-Hodgkin's lymphoma.

23. The cell according to any one of claims 20 to 22, which is a T cell.
